# EUROPÄISCHE PATENTSCHRIFT

(11) **EP 2 809 363 B1**
(45) Veröffentlichungstag und Bekanntmachung des Hinweises auf die Patenterteilung: **22.06.2022**
(21) Anmeldenummer: 13702636.5
(22) Anmeldetag: 01.02.2013
(51) Int. Cl.: A61L 15/46

(54) **WUNDPFLEGEARTIKEL, AUFWEISEND MINDESTENS EINE OBERFLÄCHE MIT ABRASIVEN EIGENSCHAFTEN**
WOUND CARE ARTICLE COMPRISING AT LEAST ONE SURFACE HAVING ABRASIVE PROPERTIES
ARTICLE POUR LE SOIN DES PLAIES COMPRENANT AU MOINS UNE SURFACE PRÉSENTANT DES PROPRIÉTÉS ABRASIVES

(30) Priorität: 01.02.2012 DE 102012100842; 10.02.2012 DE 102012101100; 17.02.2012 DE 102012101317
(43) Veröffentlichungstag der Anmeldung: 10.12.2014
(73) Patentinhaber: BSN medical GmbH, 22761 Hamburg (DE)
(72) Erfinder: RIESINGER, Birgit, 48149 Münster (DE)
(74) Vertreter: Cohausz & Florack
(86) Internationale Anmeldenummer: PCT/EP2013/052093
(87) Internationale Veröffentlichungsnummer: WO 2013/113906

(56) Entgegenhaltungen:
- WO-A1-2007/118652
- WO-A1-2010/085831
- JENNIFER HURLOW ET AL.: "Clinical experience with wound biofilm and management: a case series", OSTOMY WOUND MANAGEMENT, Bd. 55, Nr. 4, 2009, Seiten 38-49, XP002696809,

## Beschreibung

Die Erfindung betrifft einen Wundpflegeartikel gemäß dem Oberbegriff des Anspruchs 1.

Ein solcher Wundpflegeartikel ist geeignet, in der Wunde angeordnete Biofilme aufzubrechen, und/oder die Wundexsudation anzuregen.

Biofilme bestehen aus einer dünnen Schleimschicht (Film), die insbesondere saure Mucopolysaccharide enthält, und in welche der Mikroorganismen (z. B. Bakterien, Algen, Pilze, Protozoen) eingebettet sind. Biofilme entstehen, wenn Mikroorganismen an Grenzflächen durch Absonderung von Materialien eine extrazelluläre polymere Matrix ausbilden und sich in derselben ansiedeln. Sie bilden sich überwiegend in wässrigen Systemen, entweder auf der Wasseroberfläche oder auf einer Grenzfläche zu einer festen Phase, wie z.B. der Haut.

Biofilme bilden gleichsam eine Art Schirm, der die bakteriellen Biozönosen vor Einflüssen von Außen (Trockenheit, Biozide, Strahlung, etc.) schützt. Insbesondere spielen Biofilme in chronischen und akut infizierten Wunden eine große Rolle, da sie den metabolischen Austausch mit der Umwelt reduzieren, die Wundreinigung erschweren und durch ihre proteolytischen Eigenschaften sowie die ausgeschiedenen Endotoxine die Wundheilung gefährden.

Es wird geschätzt, dass die Behandlung von Biofilm-assoziierten Infektionen in den USA jährlich Kosten in Höhe von über einer Milliarde Dollar verursacht.

Biofilm-Bakterien sind gegenüber dem menschlichen Immunsystem und gegen über Antibiotikatherapien häufig weniger empfindlich.

Ursachen hierfür liegen insbesondere in einer verringerten Penetration eines Antibiotikums in die Biofilm-Matrix, sowie in der reduzierten Wachstumsrate der Bakterien im Biofilm, die sie weniger anfällig für Antibiotika macht. Weitere Ursachen sind das veränderte Mikromilieu innerhalb des Biofilms (z.B. pH- Wert, Sauerstoffgehalt), eine veränderte Genexpression, das sogenannte "Quorum sensing" (Kommunikation der Bakterien untereinander), sowie die Möglichkeit eines Gentransfers innerhalb und zwischen unterschiedlichen Bakterien. Insbesondere bei letzterem werden Plasmide von Bakterium zu Bakterium weitergegeben, die eine Antibiotikaresistenz vermitteln.

Mit dem Begriff "Wundexsudation" wird im Zuge entzündlicher Prozesse des Wundödems das Ableiten von Wundflüssigkeit aus dem Blutplasma bezeichnet. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert, wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert sind und sowohl bestehendes als auch neu gebildetes Collagen in der Wunde abbauen.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Kommt es nicht innerhalb einiger Wochen zu einer deutlichen Progression des Wundheilungsverlaufs entsprechend der verschiedenen Phasen der Wundheilung, so spricht man von einer chronischen Wunde. Dabei betrachtet man jedoch bereits länger als drei Tage andauernde exsudative Phasen als Komplikation und spricht von einer pathologischen Exsudation, welche zu einer Chronifizierung der Wunde beitragen kann. Die zugrunde liegenden Ursachen sind meist komplex und können durchaus auch systemischer Natur sein. Es überrascht jedoch aufgrund der zuvor erläuterten Bedeutung des Exsudats für die Wundheilung nicht, dass sich Komplikationen der Wundheilung in einer deutlich veränderten Zusammensetzung und Wirkung des Exsudats widerspiegeln.

Unter anderem durch eine Konzentrationsverschiebung der einzelnen Bestandteile des Exsudats verliert das normalerweise heilungsfördernde Exsudat bei chronischen Wunden seine positive Wirkung. Insbesondere der Gehalt an inflammatorischen Zytokinen und Proteasen ist in pathologischem Exsudat signifikant erhöht. Der Gehalt an Wachstumsfaktoren ist dagegen verringert. Ein besonders gravierender Unterschied ergibt sich hinsichtlich der Aktivität der zuvor angesprochenen Matrix-Metalloproteasen. Neben der Vorbereitung des Wundbetts sind sie auch beim späteren Umbau des Granulationszum Narbengewebe beteiligt. Diese Enzyme werden normalerweise als inaktives Präenzym gebildet und in ihrer Aktivierung durch entsprechende Inhibitoren reguliert (tissue inhibitors of metalloproteases, TIMPs), welche gleichzeitig selbst eine positive Wirkung auf das Zellwachstum haben. Im chronischen Exsudat scheint aufgrund von Störungen in diesem Regulationssystem die Aktivität der Proteasen erhöht, was möglicherweise zu einer Regression der Wundheilung beiträgt. Das pathologische Exsudat ist hinsichtlich des Gehalts seiner Komponenten aus dem der wundprogressionförderlichen Gleichgewicht geraten. Daraus ergeben sich verschiedene Komplikationen, die zur weiteren Verschlechterung und Chronifizierung der Wunde beitragen.

Die Anmelderin der vorliegenden Erfindung vertreibt unter dem Markenzeichen "Sorbion Sachet" eine Wundauflage enthaltend Superabsorbierende Polymere mit einem Gewichtsanteil um etwa 50 Gew.-%. Diese Wundauflage ist z.B. in der WO03094813 beschrieben und hat sich für die Behandlung von chronischen Wunden, mittelmäßig bis stark exsudierenden Wunden, die ggf. wundheilungshemmende Ödeme am Wundboden aufweisen, außerordentlich bewährt. Aufgrund des hohen Superabsorber-Anteils sind diese Wundauflagen imstande, solche chronischen Wunden aktiv zu drainieren, und dabei das Exsudat und die darin enthaltenden Bakterien und Matrix-Metalloproteasen (MMP) aufzunehmen, zu binden und zu deaktivieren.

Allerdings sind aus dem Stand der Technik bislang kaum Methoden bekannt, Biofilme in einer Wunde effektiv zu minimieren. Insbesondere aufgrund der oftmals beobachteten Antibiotikaresistenz sind Biofilme pharmazeutisch nur schwer zu behandeln. Die Anmelderin der vorliegenden Erfindung schlägt in ihrer Anmeldung DE 102007054127 die Verwendung von Phagen vor. Entsprechende Therapieansätze sind zwar äußerst vielversprechend, allerdings noch nicht bis zur klinischen Reife entwickelt, und überdies stehen derzeit auch nicht für alle in Frage kommenden Bakterien eines Biofilms spezifische Phagen zur Verfügung. Hinzu kommt, dass zur Vermeidung der Ausbildung neuer Resistenzen gegen bakteriophage Viren die Virentherapie eine Art "ultima ratio" bleiben sollte. Es ist daher wünschenswert, weitere Therapieansätze bereit zu stellen, bei welchen die Gefahr der Ausbildung von Resistenzen nicht vorliegt.

Ebenso sind aus dem Stand der Technik bislang kaum Methoden bekannt, die Exsudation einer Wunde zu erhöhen. Dies kann jedoch sinnvoll sein, z.B. um das Ausspülen unerwünschter Bestandteile (insbesondere von Restbestandteilen eines aufgelösten Biofilms, einschließlich der enthaltenen allergenen Antigene und Endotoxine) zu forcieren.

Beide Probleme - also die Auflösung von Biofilmen in einer Wunde und die Erhöhung der wundeigenen Exsudation - werden durch aus dem Stand der Technik bekannte Wundpflegeartikel, insbesondere Wundauflagen wie das oben genannte Produkt "Sorbion Sachet", nicht gelöst.

WO2010/085831 beschreibt eine Wundreinigungseinrichtung, die ein Wundreinigungstuch aufweist oder ist, welche zumindest eine Trägerschicht und an die Trägerschicht angeordnete und von der Trägerschicht abstehende Fäden, vorzugsweise ausschließlich, aus synthetischen Fasern, vorzugsweise Kunststofffasern aufweist. Die Wunde kann mit dieser Wundreinigungseinrichtung gegen eine Besiedelung mit Bakterien in Form eines flüssigen Biofilms, welcher zur Kolonisation oder lokalen Infektion der Wunde führt, behandelt werden. Besonders bevorzugt ist vorgesehen, dass zumindest einige Fäden auf ihrer von der Trägerschicht abgewandte Seite frei auskragende Enden aufweisen. Durch diese frei auskragenden Enden entwickeln die Fäden eine Art Rasierklingeneffekt.

Aufgabe der vorliegenden Erfindung ist es daher, einen Wundpflegeartikel bereit zu stellen, die die genannten Nachteile der Produkte aus dem Stand der Technik nicht aufweist. Diese Aufgabe wird mit einer Wundauflage gemäß dem vorliegenden Hauptanspruch gelöst; die Unteransprüche geben bevorzugte Ausführungsformen an. Dabei sind durch Zahlenwerte begrenzte Wertebereiche stets einschließlich der genannten Zahlenwerte zu verstehen.

Demnach ist ein Wundpflegeartikel vorgesehen, aufweisend mindestens eine Oberfläche mit abrasiven Eigenschaften, die so ausgebildet ist, dass der Wundpflegeartikel geeignet ist, bei Relativbewegung desselben zu einer Wunde
a) in der Wunde angeordnete Biofilme aufzubrechen, und/oder
b) die Wundexsudation anzuregen.

Unter dem Begriff "abrasive Eigenschaften" sollen im Folgenden solche Eigenschaften eines Materials verstanden werden, die imstande sind, die Oberfläche eines zu bearbeitenden anderen Materials, das in Kontakt mit besagtem Material steht, anzugreifen, aufzurauhen und/oder abzutragen. Dabei kann das Material mit abrasiven Eigenschaften unter Umständen vergleichbar einem Schmirgel- oder Sandpapierselbst abgetragen werden, oder aber ohne weiteren Materialverlust in seiner Form bestehen bleiben. Das zu bearbeitende Material ist diesem Fall eine Wunde, an deren Oberfläche der Wundpflegeartikel zum Reinigen oder Verbinden angesetzt, aufgelegt oder aber dauerhaft bis zum Austausch gegen einen sauberen Wundpflegeartikel angebracht wird.

Die abrasiven Eigenschaften gegenüber dem anzugreifenden, aufzurauhenden und/oder abzutragenden Material gehen zurück auf
a) eine größere Härte des abrasiven Materials im Vergleich zu dem abzutragenden Material;
b) eine große relative Oberfläche des abrasiven Materials, und/oder
c) adhäsive Eigenschaften des abrasiven Materials.

Der hier vorgestellte Wundpflegeartikel bricht mit einem Dogma der Wundversorgung, da er - im Gegensatz zu aus dem Stand der Technik bekannten Wundpflegeartikeln - der Wunde keine weiche Oberfläche zur Verfügung stellt, sondern eine Oberfläche mit abrasiven Eigenschaften. Diese Oberfläche ist imstande, in der Wunde angeordnete Biofilme aufzubrechen, und/oder die Wundexsudation anzuregen.

Die Erfinderin hat festgestellt, dass Biofilmen in der Wunde in der Regel nicht mit Antibiotika oder desinfizierenden Agenzien beizukommen ist. So werden durch besagte Mittel allenfalls die Bakterien der obersten Schicht des Biofilms abgetötet, während die darunter liegenden Schichten unbeeinträchtigt bleiben, wobei letztere u.U. noch von den Überresten der abgetöteten Bakterien profitieren.

Hingegen hat die Erfinderin erkannt, dass Biofilme - also insbesondere die die Bakterien schützende extrazelluläre Matrix - mit abrasiven Materialien aufgebrochen werden können, die im Folgenden noch beschrieben werden. Durch Entfernung des Biofilms werden die in seinem Schutze lebenden Bakterien freigelegt; sie können dann entweder mit Antibiotika oder Desinfektionsmitteln behandelt werden, oder aber sie werden durch die fortbestehende abrasive Wirkung des erfindungsgemäßen Wundpflegeartikels entfernt.

Da der genannte extrazelluläre Bio film weniger widerstandsfähig ist als der intakte Zellverband der umgebenden Haut, wird letztere durch den erfindungsgemäßen Wundpflegeartikel nicht angegriffen, während der Biofilm aufgelöst wird und die so freigesetzten Bakterien wie oben beschrieben exponiert werden.

Insbesondere vorteilhaft ist dabei, dass bei einem solchen Ansatz die Ausbildung von Resistenzen nicht zu befürchten ist.

Dieselben abrasiven Materialien sind imstande, die Wundexsudation über das normale Maß hinaus anzuregen, da sie die Wunde in einem gewünschten Maße reizen. Durch besagte forcierte Wundexsudation wird das Ausspülen von unerwünschten Fremdstoffen aus der Wunde angeregt, so z.B. von Matrix-Metalloproteasen, Mikroorganismen wie pathogenen Bakterien, Überresten der durch die abrasiven Eigenschaften aufgebrochenen Biofilme, oder bakterieller Stoffwechselprodukte und Endotoxine. Hinzu kommt, dass durch den erhöhten Anteil an Exsudat in der Wunde Nekrosen angelöst werden, die aufgrund der abrasiven Wirkung des Wundpflegeartikels anschließend abgelöst werden können. Die Wunde wird gleichsam durch taktile Reize mechanisch aufgefrischt.

Wie bereits erwähnt, kann der Wundpflegeartikel je nach Verwendung unterschiedlich ausgestaltet sein. In einer bevorzugten Ausführungsform ist der Wundpflegeartikel als Wundauflage oder Wundreinigungspad ausgeformt.

Unter Wundauflage versteht man dabei einen über einen bestimmten Zeitraum auf die Wunde platzierten Wundpflegeartikel der mit oder ohne einen zusätzlichen Verband darum verwendet wird. Demgegenüber handelt es sich bei einem Reinigungspad um einen Artikel zur Reinigung oder Desinfektion von Wunden durch entsprechend geschultes Pflegepersonal. Unter Reinigungspad ist sowohl ein Reinigungstuch als auch ein festerer Lappen in den unterschiedlichsten Größen und Dicken zu verstehen. Durch die ausgeübten Wischbewegungen wird eine abrasive Wirkung auf den Biofilm ausgeübt, die zum Aufreißendes Biofilms führt. Die Größe und Dicke des Wundpflegeartikels ist dabei der Verwendung entsprechend angepasst und entweder vorgeformt oder durch den Anwender leicht zu zuschneiden.

Der Wundpflegeartikel kann allgemein sowohl als Einwegartikel als auch zur mehrfachen Verwendung hergestellt werden.

Bei einer auf die Wunde aufgebrachte Wundauflage mit darin eingearbeiteten oder darauf aufgetragenen abrasiven Materialien, stehen diese in direktem Kontakt mit der Wunde und wirken permanent auf die Wunde ein und werden, z.B. durch Bewegungen des betreffenden Patienten, stets relativ zur Wunde bewegt. Auch in diesem Fall üben die Materialien eine abrasive Wirkung auf den Biofilm aus, die zum Aufreißen des Biofilms führt. Die im Vergleich zum Reinigungspad geringen Amplituden der genannten Relativbewegungen, und der vergleichsweise geringe Druck werden dabei durch die längere Einwirkdauer der Wundauflage kompensiert.

Die mindestens eine Oberfläche mit abrasiven Eigenschaften weist mindestens ein Material aufweisend im Wesentlichen schräg oder senkrecht zur Wundoberfläche angeordnete bzw. anordnungsfähige Mikrofasern auf.

Insbesondere kann vorgesehen sein, dass die Mikrofasern mindestens einen Fasertyp aufweisen, der ausgewählt ist aus der Gruppe enthaltend
- Polyesterfasern, wie z.B Trevira Finesse, Diolen^{®} Soft, Fortrel Microspun, DuPont Micromattique, Primabelle und/oder Shingosen,
- Polyamidfasern, wie Nylon, Timbrelle^{®}, Supplex Microfiber, Tactel^{®} Micro und/oder Silky Touch,
- Acrylfasern, wie Microsupreme, und/oder
- Polyurethanfasern.

Der Begriff "Mikrofasern" ist eine Sammelbezeichnung für Fasern, deren Einzelfäden feiner als 1 dtex sind, also ein Gewicht von maximal 1 Gramm pro 10.000 Meter aufweisen.

Aufgrund dieser Größendefinition sind Seiden- und Baumwollfasern, aber auch Flachs, Leinen, Wolle und ähnliche Naturfasern durch den Begriff "Mikrofasern" nicht erfasst. So weist feine Seide ungefähr 1,38 dtex auf, und ein Baumwollfaden weist mindestens 2 dtex auf.

In der Regel erfüllen lediglich bestimmte Kunstfasern diese Größendefinition, so z.B. Fasern aus Polyester, Polyamid, oder Acryl.

Mikrofasern sind bislang aus dem Haushaltsbereich und z.T. auch aus dem Textilbereich bekannt. Eine Verwendung in Wundpflegeartikeln wurde bislang noch nicht vorgeschlagen. Die Erfinderin der vorliegenden Erfindung hat nunmehr erstmals festgestellt, dass Mikrofasern im Vergleich zu herkömmlichen Fasern einige hervorragende Eigenschaften aufweisen, die sie für die Verwendung in Wundpflegeartikeln geeignet machen. So zeichnen sich Mikrofasern durch
a) eine große Weichheit und Bauschigkeit,
b) eine geringe Feuchtigkeitsaufnahme bei gleichzeitiger Fähigkeit, Feuchtigkeit abzuleiten,
c) eine Abrasivität gegenüber mikrobiellen Biofilmen, die durch ihre außerordentliche Feinheit und Oberflächenhärte bedingt ist,
d) eine gute Wärmeisolation, und
e) eine geringe Haftungsneigung an der Wundoberfläche
aus.

Die große Weichheit und Bauschigkeit macht Mikrofasern insbesondere geeignet für polsternde Zwecke, wie sie in der Wundpflege häufig vonnöten sind, da Wunden in der Regel stark druckempfindlich sind. Die Bauschigkeit der Fasern geht auf Formgedächtniseigenschaften zurück, die dafür sorgen, dass die Fasern nach Kompression stets in ihre ursprüngliche Form zurückkehren und so ihre polsternden Eigenschaften bewahren.

Die Tatsache, dass Mikrofasern eine geringe Feuchtigkeitsaufnahme aufweisen, reduziert z.B. die Wundrandmazeration (Aufweichung und Entzündung der umgebenden Haut durch längeren Kontakt mit einer Flüssigkeit), da mit den Wundrändern in Kontakt stehende Abschnitte eine geringe Feuchtigkeit aufweisen.

Die feuchtigkeitsleitenden Eigenschaften sind insbesondere dann von Vorteil, wenn die Mikrofasern erfindungsgemäß in Kombination mit einem Flüssigkeiten absorbierenden Abschnitt in einem Wundpflegeartikel angeordnet sind, und zwar so, dass die Mikrofasern direkt in Kontakt mit der Wunde kommen, während in einer zweiten Schicht der Flüssigkeiten absorbierende Abschnitt angeordnet ist. Auf diese Weise passiert austretendes Exsudat den Mikrofaserabschnitt (der wie eine Art Transitschicht fungiert, ähnlich wie dies von Sportunterwäsche bekannt ist) schnell und wird in den absorbierenden Abschnitt transportiert; der in Kontakt mit der Wunde stehende Mikrofasern enthaltende Abschnitt bleibt trocken.

Hinzu kommt, dass ein Wundpflegeartikel aufgrund der geringeren Feuchtigkeitsaufnahme in den Abschnitten enthaltend Mikrofasern dort im Gegensatz zu Wundpflegeartikeln mit z.B. feuchtigkeitsabsorbierenden Zellulosefasern bei Kontakt mit Flüssigkeit nicht oder weniger stark kollabiert, und so die strukturelle Integrität des Wundpflegeartikels gewahrt bleibt, was insbesondere die polsternden und wärmeisolierenden Eigenschaften aufrecht erhält.

Weiterhin wird durch die geringe Feuchtigkeitsaufnahme eine laterale Diffusion von Feuchtigkeit innerhalb des Wundpflegeartikels verhindert, was ansonsten ebenfalls zu einer Mazeration der die Wunde umgebenden gesunden Haut führen könnte.

Weiterhin wird durch die geringe Feuchtigkeitsaufnahme verhindert, dass sich innerhalb des Wundpflegeartikels ein das Wachstum von Mikroorganismen, insbesondere Bakterien, förderndes feuchtes Milieu ausbildet.

Trotz ihrer großen Weichheit weisen Mikrofasern nun eine Abrasivität gegenüber Biofilmen auf. Das heißt zum Beispiel, dass Mikrofasern, die in einen auf die Wunde aufgebrachten Wundpflegeartikel (z.B. ein Wundpflegetuch) eingearbeitet sind, bei durch einen Pfleger ausgeübten Wischbewegungen aufgrund ihrer Feinheit, die gepaart ist mit einer im Vergleich zu feuchtigkeitsabsorbierenden Zellulosefasern relativ großen Oberflächenhärte, eine abrasive Wirkung auf den Biofilm ausüben, die zum Aufreißen des Biofilms führt.

In ähnlicher Weise wirken Mikrofasern, die in eine auf die Wunde aufgebrachte Wundauflage eingearbeitet sind und mit der Wunde in Kontakt stehen, permanent auf die Wunde ein und werden, z.B. durch Bewegungen des betreffenden Patienten, stets relativ zur Wunde bewegt. Auch in diesem Fall üben die Mikrofasern aufgrund ihrer Feinheit, die gepaart ist mit einer im Vergleich zu feuchtigkeitsabsorbierenden Zellulosefasern relativ großen Oberflächenhärte, eine abrasive Wirkung auf den Biofilm aus, die zum Aufreißen des Biofilms führt. Die im Vergleich zu von einem Pfleger angewendeten Wundpflegeartikel geringen Amplituden der genannten Relativbewegungen, und der vergleichsweise geringe Druck werden dabei durch die längere Einwirkdauer der Wundauflage kompensiert.

Die pathogenen Bakterien des Films werden z.T. durch die Mikrofasern gebunden und können ggf. durch Waschen der Wunde entfernt werden. Auf diese Weise kann der metabolische Austausch der Wunde mit der Umwelt verbessert werden, es können allgemein wundheilungsfördernde Agenzien, insbesondere antibakterielle, nutritive oder proteasehemmende Agenzien, wie sie z.B. in der Anmeldung DE102007030931 der Anmelderin der vorliegenden Erfindung offenbart sind, in die Wunde eingebracht werden, und so die Wundheilung gefördert werden.

Der betreffende Patient empfindet diese abrasive Wirkung nicht als unangenehm, da er die Mikrofasern aufgrund ihrer Feinheit und ihres geringen Durchmessers als weich empfindet. Aus diesem Grunde spürt er die abrasiven Effekte nicht einmal.

Die gute Wärmeisolation geht auf die außerordentliche Feinheit und Bauschigkeit der Fasern zurück, die es ermöglicht, Luft - die als guter Wärmeisolator bekannt ist - zu immobilisieren. Grundsätzlich ist Wärme für chronische Wunden heilsam, da sie die Heilungsprozesse unterstützt. Auch aus diesem Grunde sind Mikrofasern hervorragend geeignet für die Verwendung in Wundpflegeartikeln. Hier sind insbesondere die bereits erwähnten Formgedächtniseigenschaften wichtig, die dafür sorgen, dass die Fasern nach Kompression stets in ihre ursprüngliche Form zurückkehren und so ihre polsternden Eigenschaften bewahren.

Die geringe Haftungsneigung von Mikrofasern bzw. daraus hergestellten Wundpflegeartikeln an der Wundoberfläche reduziert die schmerzintensive Wundbeeinträchtigung bei Verbandswechseln und ermöglicht so nahezu atraumatische Verbandwechsel.

Dabei ist bevorzugt vorgesehen, dass der Wundpflegeartikel eine Hülle aufweisend Mikrofasern aufweist, die bevorzugt in Form eines Gewebes, eines Gewirkes, eines Nonwovens oder eines Vlieses ausgearbeitet sind. Auf diese Weise wird der erwähnte unmittelbare Kontakt zwischen Mikrofasern und Wunde, der zu den genannten positiven Effekten führt, besonders gut gewährleistet. Ebenso kann vorgesehen sein, dass innerhalb der Hülle ein Abschnitt aufweisend Mikrofasern angeordnet ist.

Besagter Abschnitt ist aus oben genannten Gründen bevorzugt auf der der Wunde zugewandten Seite des Wundpflegeartikels angeordnet und kann beispielsweise aus einer dreidimensionalen Vliesbahn bestehen.

Der Begriff "samtartiges Material" bezeichnet ein Material, das einen mehr oder minder senkrecht zur Oberfläche angeordneten Flor aufweist. Solche Materialien werden z.B. durch Weben hergestellt. Bei der Herstellung wird ein zweiter Schuss- oder Kettfaden eingearbeitet. Dieser Faden bildet Schlaufen (Flottungen), welche auf der einen Seite den charakteristischen Faserflor ergeben, nachdem sie aufgeschnitten worden sind. Der abrasive Effekt dieses Materials kommt - ähnlich wie bei Borsten - durch die relativen Bewegungen der im Wesentlichen rechtwinklig zur Wundoberfläche angeordneten Florfasern bei Anordnung des Wundpflegeartikels auf der Wunde zustande.

Ein solches Material wird aus den oben erwähnten Mikrofasern hergestellt.

Bevorzugt ist besonders vorgesehen, dass das erfindungsgemäße Produkt mindestens ein Detergenz enthält.

Detergenzien besitzen oberflächenaktive Eigenschaften und sind insbesondere in der Lage, Mucopolysacchardschleime zu lösen. Auf diese Weise können die abrasiven, Bio film aufbrechenden Eigenschaften des erfindungsgemäßen Wundpflegeartikels unterstützt werden. Bei besagten Detergenzien handelt es sich bevorzugt um nicht toxische, physiologisch akzeptable Detergenzien, wie z.B. Pluronic. Letztere können insbesondere in trockener Form in die besagte Matrix (z.B. die Borsten) eingebracht und bei Kontakt mit Feuchtigkeit mobilisiert werden. Nach einiger Verweildauer des Wundpflegeartikels wird die Exsudation angeregt und Exsudat gelangt in den Wundpflegeartikel, wo es die besagten Detergenzien verfügbar macht, die ihrerseits dann eine aufbrechende Wirkung auf den Biofilm ausüben.

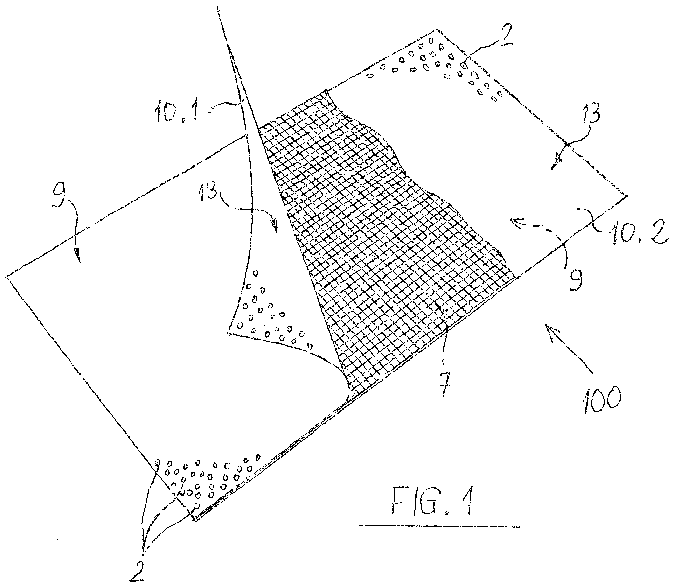

In einer bevorzugten Ausführungsform ist vorgesehen, dass der Wundpflegeartikel einen Materialabschnitt mit abrasiven Eigenschaften aufweist, der gegenüber dem übrigen Wundpflegeartikel frei schwimmend angeordnet ist, dergestalt, dass er im auf den Körper des Patienten aufgelegten Zustand seine Lage gegenüber dem übrigen Wundpflegeartikel bzw. gegenüber der Wunde ändern kann (siehe Fig. 1). Hierzu eignet sich insbesondere das zuvor schon beschriebene dreidimensionale Wunddistanzgitter.

Ferner ist besonders bevorzugt vorgesehen, dass besagter Materialabschnitt in einer viskosen Schicht schwimmt. Die besagte viskose Schicht ist bevorzugt zwischen dem besagten Materialabschnitt und dem Rest des Wundpflegeartikels angeordnet. Dabei kann es sich z.B. um eine Schicht aus Paraffin, Vaseline, oder Bienenhonig handeln.

Die meisten Honigarten verfügen über eine antibakterielle Wirkung, die auf das bei Kontakt mit Wasser freigesetzte Wasserstoffperoxid (H₂O₂) zurückgeht und als Hyperoxyd-Tätigkeit oder PA bezeichnet. Ursache hierfür ist die Aktivität des Enzyms Glukose-Oxydase im Honig. Überdies hat Honig hygroskopische Eigenschaften, die die Wundexsudation anregen. Besagter Honig weist hier also eine Doppelfunktion auf: Einerseits dient er als hochviskoses Gleitmittel zwischen abrasivem Materialabschnitt und dem Rest des Wundpflegeartikels, und andererseits weist er eine desinfizierende und die Exsudation fördernde Wirkung auf.

Besonders bevorzugt handelt es sich dabei um Manuka-Honig, also Honig, der überwiegend aus der Manuka- und der Kanuka-Pflanze gewonnen wird und gegenüber herkömmlichen Honigen einige Vorteile aufweist. So wird die besagte Glukose-Oxydase durch Licht und Hitze deaktiviert. Die antibakterielle Wirkung des Manuka-Honigs geht im Gegensatz dazu auf Hyperoxyd zurück, das beständiger ist und nicht durch Licht und/oder mäßige Hitze beeinflusst wird.

Wie bereits erwähnt ist eine Wundauflage eine bevorzugte Ausführungsform des Wundpflegeartikels. Die Wundauflage z.B. flächig ausgebildet sein und eine runde, ellipsoide, rechteckige oder polygonale Form aufweisen. Ebenso kann es sich bei dem Wundpflegeartikel aber auch um einen Artikel zur Reinigung oder Desinfektion der Wunde handeln. Neben Wundpflegetuch oder Reinigungspad kommen dabei auch Tupfer, Tampon, eine Wundtamponade oder dergleichen als Ausführungsformen in Frage. Diese haben absorbierende Eigenschaften.

In einer weiteren Ausführungsform ist vorgesehen, dass der Wundpflegeartikel eine mindestens abschnittsweise um den Wundexsudate absorbierenden

Körper angeordnete Hülle aufweist. Die Hülle kann sowohl geschlossen sein als auch als Umschlag mit überlappenden Bereichen ausgeformt sein. Dabei kann sowohl eine umlaufende Naht als auch eine nahtfreie Ausführungsform gewählt werden. Die Hülle kann den Absorptionskörper sowohl komplett als auch lediglich in Teilen einfassen, oder aber nur auf der der Wunde zugewandten Seite fixiert oder lose schwimmend aufgelegt werden.

In dieser Ausführungsform ist die abrasive Oberfläche bevorzugt in einem Abschnitt der Hülle angeordnet. In einer Ausführungsform ist der Absorptionskörper lediglich auf der Wunde zugewandten Seite mit dem abrasiven Hüllenmaterial unterlegt. Ganz besonders bevorzugt ist hier vorgesehen, dass die Hülle ganz oder teilweise aus dem bereits beschriebenen dreidimensionalen Wunddistanzgitter besteht.

Dabei weist der Wundexsudate absorbierende Körper mindestens ein Material auf, das ausgewählt ist aus der Gruppe enthaltend eine Matte, insbesondere aus einem Airlaid aus besagten Garnen oder Fasern aus superabsorbierenden Polymeren mit eingearbeiteten superabsorbierenden

Polymeren. Besagte Airlaidmatte kann bevorzugt einen im Wesentlichen flachen Materialabschnitt aus Absorptionsmaterial aufweisen, der z. B. aus einem aufsaugenden Vlies aus den genannten Fasern mit darin verteilten superabsorbierenden Polymeren besteht.

Dieser Wundexsudate absorbierende Körper kann der absorbierenden Einlage entsprechen, die in einer Wundauflage der Anmelderin der vorliegenden Erfindung enthalten ist, wie sie beispielsweise in der WO03094813, der WO2007051599 und der WO0152780 offenbart ist und unter dem Handelsnamen "sorbion Sachet" vertrieben wird.

Der Wundexsudate absorbierende Körper kann in einer anderen Ausgestaltung ebenso einen Kern bilden, der - ggf. flockenartige - Fasern oder Garne aus superabsorbierenden Polymeren sowie superabsorbierenden Polymeren in Granulatform aufweist, wobei die Granulate an die Fasern bzw. Garne in mehreren Höhen angeklebt bzw. angeschweißt sind, und die Granulate über mehr als 50 % der gesamten Bauhöhe wenigstens eines Abschnitts des Kerns verteilt sind, wobei vermengte Bereiche von Granulat und Fasern vorliegen. Der Gewichtsanteil der superabsorbierenden Polymeren kann dabei bevorzugt im Bereich zwischen 10 - 25 Gew.-% liegen. Ähnliche Konstruktionen sind aus herkömmlichen Inkontinenzmaterialien bekannt und wie Hygienebinden für ihre polsternden Eigenschaften bekannt.

Für den absorbierenden Körper kommen ebenso die bereits zuvor genannten Mikrofasern in Frage. Diese können sowohl alleine als auch in Kombination mit anderen Fasern sowie den superabsorbierenden Polymeren verwendet werden.

Um besagten Kern herum kann eine Hülle angeordnet sein, die in Bereichen überlappend angeordnet ist, und der z.B. eine Klebenaht überdeckt bzw. Teil derselben ist.

Ebenso kann innerhalb der Hülle ein Abschnitt eines hydrophoben und/oder wasserabweisenden bzw. wasserundurchlässigen Materials vorgesehen sein, der Durchnässungs- oder Wäscheschutz fungiert.

Der Wundexsudate absorbierende Körper kann in einer anderen Ausgestaltung ebenso mindestens eine flache Lagen aufweisend Fasern oder Garne aus superabsorbierenden Polymeren enthalten, an welche superabsorbierende Polymere in Granulatform geklebt sind. Dadurch ergibt sich in einer bevorzugten Ausgestaltung ein Aufbau des Körpers, der wenigstens drei Schichten aufweist, wobei zwei Deckschichten eine Schicht aufweisend superabsorbierende Polymere umgeben.

Dabei liegen in der Ebene keine Vermengungen von Fasern und superabsorbierenden Polymeren vor; sondern lediglich fixierte Benachbarungen beider Materialien. Die ggf. vorgesehenen mehreren Lagen können dabei in einer bevorzugten Ausgestaltung auch durch walzen, Pressen, Kalandrieren oder ähnliche Verfahren physisch miteinander verdichtet sein. Überdies kann der Körper sich wiederholende Musterungen oder Maserungen aufweisen, wie z.B. ein Karomuster, ein Stanzmuster oder dergleichen.

In einer besonderen Ausführungsform ist vorgesehen, dass der Wundpflegeartikel, insbesondere der Wundexsudate absorbierende Körper, superabsorbierende Polymere aufweist. Diese können bei einer Wundauflage in die absorbierenden Körper und/oder in die Hülle eingearbeitet sein, oder aber als loser Bestandteil innerhalb der Hülle platziert werden. Letztere Anordnung bedingt eine der Größe der superabsorbierenden Polymere angepasste Hülle, bei der die superabsorbierenden Polymere nicht aus der Hülle hinaus rieseln. Wie noch weiter ausgeführt wird, sind sowohl superabsorbierende Partikel oder aber Fasern möglich, die entweder als lose Schüttung vorliegen oder aber in das umgebende Material eingearbeitet sind. Auch bei einem Wundreinigungspad ist die Fixierung der superabsorbierenden Polymere der Ausgestaltung des Pads oder Tuches anzupassen.

Superabsorbierende Polymere (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in das Polymerpartikel quillt es auf und strafft auf molekularer Ebene dieses Netzwerk, so dass das Wasser ohne Hilfe nicht mehr entweichen kann.

Die Verwendung von SAP in Wundauflagen zur Aufnahme von Exsudat ist bereits aus der WO0152780 und der WO03094813 der Anmelderin der vorliegenden Erfindung bekannt.

Der Begriff "Exsudat" bezeichnet eine über die entzündlichen Prozesse des Wundödems vom Blutplasma abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert, wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert sind und sowohl bestehendes als auch neu gebildetes Collagen in der Wunde abbauen.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Kommt es nicht innerhalb einiger Wochen zu einer deutlichen Progression des Wundheilungsverlaufs entsprechend der verschiedenen Phasen der Wundheilung, so spricht man von einer chronischen Wunde. Dabei betrachtet man jedoch bereits länger als drei Tage andauernde exsudative Phasen als Komplikation und spricht von einer pathologischen Exsudation, welche zu einer Chronifizierung der Wunde beitragen kann. Die zugrunde liegenden Ursachen sind meist komplex und können durchaus auch systemischer Natur sein. Es überrascht jedoch aufgrund der zuvor erläuterten Bedeutung des Exsudats für die Wundheilung nicht, dass sich Komplikationen der Wundheilung in einer deutlich veränderten Zusammensetzung und Wirkung des Exsudats widerspiegeln.

Unter anderem durch eine Konzentrationsverschiebung der einzelnen Bestandteile des Exsudats verliert das normalerweise heilungsfördernde Exsudat bei chronischen Wunden seine positive Wirkung. Insbesondere der Gehalt an inflammatorischen Zytokinen und Proteasen ist in pathologischem Exsudat signifikant erhöht. Der Gehalt an Wachstumsfaktoren ist dagegen verringert. Ein besonders gravierender Unterschied ergibt sich hinsichtlich der Aktivität der zuvor angesprochenen Matrix-Metalloproteasen. Neben der Vorbereitung des Wundbetts sind sie auch beim späteren Umbau des Granulationszum Narbengewebe beteiligt. Diese Enzyme werden normalerweise als inaktives Präenzym gebildet und in ihrer Aktivierung durch entsprechende Inhibitoren reguliert (tissue inhibitors of metalloproteases, TIMPs), welche gleichzeitig selbst eine positive Wirkung auf das Zellwachstum haben. Im chronischen Exsudat scheint aufgrund von Störungen in diesem Regulationssystem die Aktivität der Proteasen erhöht, was möglicherweise zu einer Regression der Wundheilung beiträgt. Das pathologische Exsudat ist hinsichtlich des Gehalts seiner Komponenten aus dem der Wundprogression förderlichem Gleichgewicht geraten. Daraus ergeben sich verschiedene Komplikationen, die zur weiteren Verschlechterung und Chronifizierung der Wunde beitragen.

Im Zusammenhang mit dem erfindungsgemäßen Wundpflegeartikel aufweisend mindestens eine Oberfläche mit abrasiven Eigenschaften, der imstande ist, in der Wunde angeordnete Biofilme aufzubrechen, und/oder die Wundexsudation anzuregen, kommt der besagten Komponente aufweisend SAP eine besondere Bedeutung zu.

So dient der vorgesehene Anteil an SAP dazu, die erzeugten Bruchstücke und Überreste des Biofilms aufzunehmen. Durch die Zerstörung des Biofilms werden ggf. auch Endotoxine und bakterielle Pathogenitätsfaktoren (insbesondere bakterielles Hämolysin und Leukocidin) freigegeben, die beim Patienten Entzündungen, Allergien, Schocks (insbesondere anaphylaktische Schocks und/oder das Toxic Shock Syndrome) und Fieber erzeugen können (Herxheimer-Reaktion). Besagte Endotoxine und Pathogenitätsfaktoren werden durch den Anteil an SAP aufgenommen, so dass die besagten Folgen vermieden können.

In der DE102007054127 der Anmelderin der vorliegenden Erfindung wird auf diese Verhältnisse genau eingegangen; insbesondere wird gezeigt, dass SAP in der Lage sind, Bakterien und bakterielle Endotoxine zu binden.

Überdies dient der vorgesehene Anteil an SAP dazu, die erhöhte Menge an Exsudat, die durch Verwendung des erfindungsgemäßen Wundpflegeartikels erzeugt wird, aufzunehmen. Dabei ist besonders bevorzugt vorgesehen, dass der Wundexsudate absorbierende Körper ein Muster aus Inzisionen und/oder Stanzungen aufweist. Diese sind bevorzugt dergestalt ausgebildet und/oder angeordnet, dass sie den Flüssigkeitseintritt in den Wundpflegeartikel erleichtern. Dieses Merkmal entfaltet besondere Vorteile im Zusammenspiel mit der Hülle aus einem dreidimensionalen Folienmaterial mit nach außen bzw. zur Wunde gekehrten Öffnungen bzw. Perforationen, die dem Wundpflegeartikel eine rauhe Außenfläche und damit abrasive Eigenschaften verleihen, da so eine effektive und schnelle Aufnahme des Exsudats, dass durch Verwendung des erfindungsgemäßen Wundpflegeartikels erzeugt wird, zu gewährleisten.

Auf diese Weise ergänzen sich die superabsorbierenden Polymere und die Stanzungen und Inzision des Wundexsudate absorbierende Körpers bzw. des Wundpflegeartikels aufweisend mindestens eine Oberfläche mit abrasiven Eigenschaften synergistisch.

Bevorzugt weisen die superabsorbierenden Polymeren eine Pulver-, Granulat-, Faser-, Garn-, Watte-, Vlies-, und/oder Gewebeform auf.

Besonders bevorzugt weisen die superabsorbierenden Polymeren eine Pulver- oder Granulatform auf. Superabsorbierende Polymere weisen in dieser Darreichungsform eine Vielzahl von Vorteilen auf uns sind z.B. aus der WO0152780 und der WO03094813 der Anmelderin der vorliegenden Erfindung bekannt.

Ganz besonders bevorzugt weisen die superabsorbierenden Polymeren eine Faser-, Garn-, Watte-, Vlies- oder Gewebeform auf. Hier wird auf die DE 102007049429 der Anmelderin der vorliegenden Erfindung verwiesen.

Die superabsorbierenden Polymere können jedoch auch in jeglicher Kombination aus verschiedenen Formen im Wundpflegeartikel vorliegen.

Die vorgeschlagene Verwendung von Superabsorbierenden Polymeren in Faser- oder Garnform weist gegenüber partikulären superabsorbierenden Polymeren eine Reihe von Vorteilen auf:
i) So haben besagte Fasern oder Garne einen Dochteffekt. Auf diese Weise können sie bei Kontakt mit einer Flüssigkeit sehr viel schneller diese Flüssigkeit aufnehmen und binden als dies partikuläre superabsorbierende Polymere können.
ii) überdies können die Flüssigkeitsströme lenken bzw. richten. Auf diese Weise kann z.B. eine Wundrandmazeration verhindert werden.
iii) Sie können anders als partikuläre superabsorbierende Polymere zu Vliesen, Geweben oder ähnlichem verarbeitet werden. Auf diese Weise kann z.B. die Hülle einer Wundauflage aus diesen Fasern oder Garnen gefertigt sein, und die superabsorbierenden Eigenschaften können so sehr viel näher an den Wundgrund gebracht werden.
iv) Die Gefahr, dass superabsorbierende Materialien in der Wunde verbleiben, ist bei einem Gewebe aus superabsorbierenden Garnen sehr viel geringer als bei partikulären superabsorbierenden Polymeren. Auch dies trägt dazu bei, dass die superabsorbierenden Eigenschaften so sehr viel näher an den Wundgrund gebracht werden können
v) Da in vielen Fällen auf ein gesondertes Trägermaterial verzichtet werden kann, kann der Anteil an superabsorbierenden Materialien in einem Hygiene- oder Pflegeartikel, insbesondere einem Wundpflegeartikel erheblich erhöht werden; im Extremfall kann er sogar einen Gewichtsanteil von 100 % annehmen.
vi) Besagte Fasern oder Garne bzw. die daraus hergestellten Produkte besitzen eine sehr viel höhere Weichheit und eine geringere Abrasivität als die entsprechenden partikulären superabsorbierenden Polymere.
vii) Besagte Fasern oder Garne lassen sich zu einem Gefüge verarbeiten, ohne dass - wie es bei partikulären superabsorbierenden Polymeren erforderlich ist - ein Kleber oder ein Schweissverfahren verwendet werden muß. Dies hat sowohl in Bezug auf die Reinheit des Produkts als auch in Bezug auf die Pharmakologie und etwaige Allergenität erhebliche Vorteile.
viii) Im Gegensatz zu partikulären superabsorbierenden Polymere läßt sich die Dimensionierung besagter Fasern oder Garne sehr viel genauer steuern und kontrollieren, was einerseits zu Verhinderung von Stäuben führt, wie sie bei Verwendung von partikulären superabsorbierenden Polymere häufig entstehen., und was andererseits die Produktqualität (Homogenität und Reproduzierbarkeit) wesentlich erhöht.
ix) Aufgrund der fehlenden Ausbildung von Stäuben kann ggf. auch auf die Verwendung einer gesonderten Hülle verzichtet werden.
x) Besagte Garne oder Fasern können in aus dem Stand der Technik bekannten Airlaids, die partikuläre superabsorbierenden Polymere enthalten, die Trägerfasern des Airlaids ersetzen, um so den Anteil an superabsorbierenden Materialien in einem Hygiene- oder Pflegeartikel, insbesondere einem Wundpflegeartikel und damit die gesamte Absorptionskapazität - zu erhöhen.

Jegliche zwei- oder dreidimensionale Anordnung der Fasern oder Garne ist hier denkbar. So können die Fasern oder Garne gerichtet oder ungerichtet (Wirr-Warr), in mehreren Lagen oder sonst wie angeordnet sein.

Superabsorbierende Fasern aus Polyacrylaten werden z.B. von der Firma Technical Absorbents unter dem Handelsnamen "Oasis Super Absorbent Fibre" angeboten und vertrieben. Sie weisen wie alle Superabsorbierenden Polyacrylate eine sehr hohe Aufnahmekapazität für Flüssigkeiten auf. Diese Fasern können z.B. in Form eines Vlieses, eines Airlaids, eines Gewebes, eines Airlaids und/oder eines Nonwoven vorliegen. Solche Fasern sind z.B. aus der DE 69807337 bekannt.

Die besagten Fasern aus superabsorbierendem Material sind jedoch in der Regel sehr brüchig, da das verwendete Polyacrylat-Material eine hohe Sprödheit aufweist. Aus diesem Grunde können die Fasern eine gewisse Länge nicht überschreiten und daher auch nicht ohne weiteres zu einem Garn verarbeitet werden.

Die WO0106047 beschreibt hingegen Garne aufweisend superabsorbierende Polymere. Diesen Garnen liegt ein besonderes Herstellungsverfahren zugrunde, bei welchem die oben genannten superabsorbierenden Fasern mit stützenden Fasern aus einem stärkeren Material gemischt und anschließend zu einem Garn versponnen werden. Bei besagten stützenden Fasern handelt es sich z.B. um Fasern aus Polyester, Polypropylen, Nylon, Baumwolle, Viskose oder ähnlichem Material. Das so hergestellte Garn kann dann zu einem Gewebe, Gelege, Gestrick und/oder Gewirke verarbeitet werden. Ebenso können die Fasern aber auch mit elastischen Fasern, beispielsweise aus Elasthan verarbeitet werden.

Bislang ist jedoch ausschließlich die Verwendung solcher Garne für Kabel, insbesondere Unterwasserkabel, beschrieben, so z.B. in der EP1072698, die vom selben Anmelder stammt wie die oben diskutierte WO0106047.

Bevorzugt ist dabei vorgesehen, dass die in Faserform vorliegenden superabsorbierenden Polymere mindestens teilweise in Form einer Watte, eines Vlieses, eines Airlaids und/oder eines Nonwoven vorliegen.

Hierzu werden bevorzugt Fasern mit mittleren Längen von 5 - 50 mm verwendet. Die Herstellung erfolgt mit bekannten Methoden, wie z.B. dem Kardieren oder dem Airlaid-Verfahren. Dabei können die Superabsorbierenden Fasern alleiniger Bestandteil des jeweiligen Materials sein. Bevorzugt ist jedoch vorgesehen, dass der Hygiene- oder Pflegeartikel überdies einen Anteil an stützenden Fasern aufweist, die auch in feuchtem Zustand die Integrität der Wundauflage gewährleisten, wobei besagte Fasern ausgewählt sind aus der Gruppe enthaltend
a) Cellulosefasern,
b) Viskosefasern,
c) Alginatfasern, und/oder
d) Polyester-, Polyolefine-, Polyurethan-, Polyvinylalkohol- oder Polyamidfasern, bzw. Mischungen oder Copolymere derselben.

Auf diese Weise lassen sich die Absorptionseigenschaften des betreffenden Materials, aber auch andere Eigenschaften wie z.B. Dehnbarkeit, Reißfestigkeit, Verhalten bei Durchnässung und dergleichen, genau steuern.

Dabei kann insbesondere vorgesehen sein, dass ein Gewebe vorgesehen ist, bei dem z.B. die Kettfäden aus superabsorbierenden Garnen gefertigt sind, während die Schussfäden aus anderen Garnen bestehen, die z.B. stützende Fasern gemäß der obigen Aufzählung enthalten. Ein solches Gewebe weist gerichtete hydroaktive Eigenschaften auf, d.h. es nimmt Flüssigkeit in einer Richtung auf und leitet sie weiter.

Bevorzugt können die Fasern mindestens teilweise in Form eines Vlieses, eines Gewebes, eines Gewirkes, eines Airlaids und/oder eines Nonwoven vorliegen.

Weiterhin kann vorgesehen sein, dass die Mikrofasern zumindest abschnittsweise in einem Verbund mit anderen Fasern vorliegen.

Hier ist z.B. an Fasern aus Cellulose, aus superabsorbierenden Polymeren und/oder Fasern aus modifizierter Zellulose, insbesondere Carboxymethylcellulose (CMC), gedacht. Ein solcher Verbund würde Teile der genannten positiven Eigenschaften der Mikrofasern (Abrasivität, geringe Feuchtigkeitsaufnahme), gleichwohl aber die polsternden und wärmeisolierenden Eigenschaften der Mikrofasern bewahren.

So weist ein Gemisch aus Cellulose und Mikrofasern einen Dochteffekt auf und verbessert so die feuchtigkeitsleitenden Eigenschaften.

Ein Gemisch aus CMC bzw. Fasern aus superabsorbierenden Polymeren und Mikrofasern wandelt sich bei Feuchtigkeitsaufnahme partiell in ein Gel um, bewahrt dabei aber seine strukturelle Integrität. Hier wird auf die Patentanmeldungen DE 102007049429 und DE 102007049430 der Anmelderin der vorliegenden Erfindung verwiesen.

Ferner ist bevorzugt vorgesehen, dass das Material der Hülle mindestens einen hydrophoben und/oder nicht mit der Wunde verklebenden Anteil aufweist.

Ein solches Material verhindert wirksam, dass die Wundauflage mit der Wunde verklebt, und reduziert so Traumata beim Verbandwechsel. Hierbei kann es sich beispielsweise um Baumwolle, insbesondere mercerisierte Baumwolle, regenerierte Zellulose wie Viskose, Lyocell, Modal, Rayon oder Kupferseide, Polyamid (Nylon), Polyethylen, Polypropylen, PTFE (Teflon), silikonisierte Fasern oder ähnliche Materialien handeln. Insbesondere kann es sich auch um Mikrofasern handeln.

Ebenso kann es sich bei diesem Material um ein gelbildendes Material handeln, wie z.B. modifizierte gelbildende Zellulosen wie Carboxymethylcellulose (CMC), ein Compound aus Nylon, CMC und Vaseline, oder ein Compound aus CMC und Cellulosefasern. Eine solche Hülle ist z.B. in der DE 102007049430 der Anmelderin der vorliegenden Erfindung beschrieben.

In einer anderen bevorzugten Ausgestaltung kann die Hülle aus einer Schicht enthaltend superabsorbierende Polymere in Faserform aufweisen. Ein solche Hülle ist z.B. in der DE 102007036758 der Anmelderin der vorliegenden Erfindung beschrieben.

Besonders bevorzugt weist das Material dabei eine dreidimensionale Porenstruktur auf, wie sie z.B. in der WO2007118652 der Anmelderin der vorliegenden Erfindung beschrieben ist.

Besagte Hülle kann bevorzugt aus einem Nonwoven aus Polypropylen mit einem Flächengewicht von > 10 bis < 100 g/m², bevorzugt von > 20 bis < 40 g/m², bestehen.

Ebenso kann innerhalb der Hülle ein Abschnitt eines hydrophoben und/oder wasserabweisenden bzw. wasserundurchlässigen Materials vorgesehen sein, der als Durchnässungs- oder Wäscheschutz fungiert.

Bevorzugt ist insbesondere vorgesehen, dass die verwendeten superabsorbierenden Polymere im Wundpflegeartikel immobilisiert sind. Dies ist wichtig, um ein Herausrieseln der Polymere aus der Wundauflage in die Wunde zu verhindern. Wie bereits dargestellt, können die superabsorbierenden Polymere dazu sowohl in das Material des Wundpflegeartikels eingearbeitet sein, oder aber durch Aufnahme innerhalb einer das Material umgebenden Hülle vor dem Herausrieseln geschützt werden.

Besagte Immobilisierung kann auf verschiedene Arten erfolgen. So kann vorgesehen sein, dass
a) die superabsorbierenden Polymere mit Fasern der Wundauflage verklebt sind, z.B. mit einem Hotmelt-Kleber;
b) die superabsorbierenden Polymere mit Fasern der Wundauflage verpresst sind;
c) die superabsorbierenden Polymere fest in eine dreidimensionale Fasermatrix eingebettet sind;
d) der die superabsorbierenden Polymere enthaltende Abschnitt der Wundauflage von einer Hülle umgegeben ist, die - z.B. durch Wahl einer geeigneten Porengröße oder durch Verzicht auf Poren - undurchlässig ist für die Polymere (hierbei kann es sich um die Außenhülle der Wundauflage handeln, jedoch auch um eine innerhalb der Außenhülle angeordnete innere Hülle);
e) die superabsorbierenden Polymere in Faserform vorliegen, wobei die Fasern in Form eines Faserverbundes vorliegen und ggf. sogar mit den Zellstofffasern der Wundauflage einen Verbund eingehen.

In einer bevorzugten Ausführungsform weist das Material der Hülle keine willentlich eingebrachten Poren auf, hier sind Poren im Material allenfalls durch die Maschenweite des Gewebes oder den Faserabstand des Vlieses gegeben. In einer anderen bevorzugten Ausführungsform weist das Material der Hülle willentlich eingebrachte Poren auf, die Porengröße richtet sich dabei nach der Größe der verwendeten Superabsorber-Teilchen, und kann dabei Werte von < 50 bis < 5000 µm annehmen, bevorzugt < 100 bis < 3000 µm.

Weiterhin ist bevorzugt vorgesehen, dass der Wundpflegeartikel eine an anatomische Gegebenheiten angepasste Form aufweist. Im Falle einer Wundauflage kann diese z.B. in Form einer Manschette ausgebildet sein, die über den einen Arm oder ein Bein oder ein Gelenk gestülpt werden kann, oder in Form eines an die Ferse, das Ellenbogengelenk oder dergleichen angepassten Verbandes.

Weiterhin ist bevorzugt vorgesehen, dass der Wundpflegeartikel weiterhin aufweist
a. einen Abschnitt aufweisend Weichschaumstoff
b. einen Abschnitt aufweisend modifizierte Cellulose, insbesondere Carboxymethylcellulose, und/oder
c. einen Abschnitt aufweisend Alginate, und/oder
d. einen Abschnitt aufweisend ein Vlies und/oder eine Airlaidmatte, oder Schnipsel derselben.

Der Begriff "Airlaid" bezeichnet einen speziellen Vliesstoff aus Zellstoff und Polyolefinfasern, in den ggf. superabsorbierende Polymere eingebettet sind. Hierbei kann es sich z.B. um die im Produkt "Sorbion Sachet" enthaltene Airlaidmatte, die in der WO03094813 der Anmelderin der vorliegenden Erfindung beschrieben ist, handeln.

Dabei kann dem Airlaid ein Anteil an Zellstoff über- oder unterlagert sein, z.B. ein Anteil an Watte, Fluff pulp, medizinische Watte, Zellwolle, Zellstoffflocken, regenerierte Zellulose, Schnipsel, Rayon, Viskose, Modal und/oder Tencel, wobei besagter Anteil zusätzlich zum Airlaid oder aber exklusiv einen Anteil an superabsorbierenden Polymeren enthält.

Der Begriff "Vlies" bezeichnet ein textiles Flächengebilde aus einzelnen Fasern, das im Gegensatz zu Geweben, Gestricken und Gewirken nicht aus Garnen hergestellt wird. Vliese behalten ihre strukturelle Integrität i.d.R. durch Haftung der einzelnen Fasern aneinander. Sie werden auch als "Nonwovens" bezeichnet, und z.B. durch Walken der Fasern hergestellt

Bei besagtem Schaum kann es sich z.B. um ein Material ausgewählt aus der Gruppe enthaltend thermoplastische Weichschäume, wie Polyurethan-, Polyamid- oder Polyetherschaum, Silikonschaum sowie Cellulose-Schaum oder Naturschwamm handelt. Besagter Schaum kann bevorzugt formstabile Eigenschaften aufweisen.

Naturschwämme, beispielsweise der Klasse der Hornkieselschwämme (Demospongiae), weisen ähnlich wie technische Schaumstoffe ein Absorptionsvermögen für Flüssigkeiten auf. Überdies weisen sie wachstumshemmende Eigenschaften gegenüber Mikroorganismen auf, um sich vor dem Ansiedeln von sessilen Organismen zu schützen. Diese Eigenschaften können auch in Zusammenhang mit der Wundversorgung sinnvoll sein, um Bakterienwachstum im Wundpflegeartikel und/oder in der Wunde zu verhindern. Ebenso weisen diese Schwämme wachstumshemmende Eigenschaften gegenüber Pilzen und Einzellern auf. Hinzu kommt, dass solche Schwämme Flüssigkeiten zu absorbieren imstande sind und sich daher für die Aufnahme von Exsudaten hervorragend eignen.

Besagter Naturschwamm kann in dünnen Scheiben, die z. B. durch thermisches Schneiden erzeugt worden sind, auf die Wunde aufgelegt sein.

Der Weichschaumstoff kann ggf. mehrlagig ausgestaltet sein, wobei die einzelnen Lagen bevorzugt Dicken zwischen 0,5 mm und 10 mm aufweisen können.

Der Weichschaumstoff kann offenzellig und geschlossenzellig ausgestaltet sein. Insbesondere in letzterem Falle kann vorgesehen sein, dass das Material Stanzungen und/oder Perforationen aufweist, welche den Flüssigkeitsein- und -durchtritt in bzw. durch den Weichschaumstoff beschleunigen. Überdies kann der Weichschaumstoff auch als Integralschaum ausgestaltet sein.

Alginate werden aus den Braunalgen gewonnen und zu einem faserigen Vlies verwoben Chemisch handelt es sich um Polysaccharide, und zwar Calcium- und/oder Natriumsalze der Alginsäuren. Alginate können bis zum 20-fachen ihres Eigengewichtes an Flüssigkeit aufnehmen, dabei wird das Wundexsudat in die Hohlräume eingelagert. Die im Alginatgitter enthaltenen Ca²⁺ -Ionen werden gegen die Na+ -Ionen aus dem Exsudat ausgetauscht, bis der Sättigungsgrad an Na-Ionen im Alginat erreicht ist. Dabei kommt es zu einem Aufquellen der Wundauflage und zur Umwandlung der Alginatfaser in einen Gelkörper durch Aufquellen der Fasern.

Carboxymethylcellulose liegt insbesondere in Form von Natriumcarboxymethylcellulose vor und ist unter dem Namen "Hydrofaser" im Handel. In Hygiene- und Wundprodukten werden die Fasern in eine flächige Matrix überführt. Durch die Aufnahme von Flüssigkeit aus dem Wundexsudat werden die Fasern nach und nach in ein Gelkissen umgewandelt, das die Flüssigkeit hält und nicht wieder freigibt. Dabei sind die Fasern so aufgebaut, dass das Wundexsudat nur in vertikaler Richtung aufgenommen wird. Dies bedeutet, dass, solange die Kapazität reicht, das Exsudat nicht über den Wundrand fließt. Auf diese Weise kann eine Wundrandmazeration effektiv verhindert werden.

All die oben unter den Punkten a - d genannten Materialien bzw. Abschnitte können in Sandwichanordnung auf bzw. unter dem genannten Wundexsudate absorbierenden Körper aufweisend einen Anteil an Zellstoff angeordnet sein. Es kann jedoch ebenso vorgesehen sein, dass erstere in Form einer Einlage in den Wundexsudate absorbierenden Körper integriert sind, z.B. in Form eines Streifens, eines runden Abschnitts oder eines kreuzförmigen Abschnitts. Hierbei bildet der Wundexsudate absorbierenden Körper ein entsprechend ausgeformtes Fenster oder zumindest eine einseitige Aussparung. Umgekehrt kann vorgesehen sein, dass einer der unter den Punkten a - d genannten Abschnitte ein Fenster oder zumindest eine einseitige Aussparung ausbildet, in welcher der Wundexsudate absorbierende Körper aufweisend einen Anteil an Zellstoff angeordnet ist. Weiterhin ist bevorzugt vorgesehen, dass zwischen der Hülle und dem Wundexsudate absorbierenden Körper wenigstens ein flüssigkeitsundurchlässiger Wäscheschutz-Folienabschnitt angeordnet ist. Dieser kann bevorzugt so angeordnet sein, dass er nur den mittleren Bereich des Wundpflegeartikels abdeckt.

Weiterhin ist bevorzugt ein Kit für die Akut-, Notfall- oder Militärmedizinische bzw. die chronische Versorgung vorgesehen, aufweisend einen Wundpflegeartikel gemäß einem der vorherigen Ansprüche.

Bevorzugt weist ein solches Kit einen Wundpflegeartikel gemäß einem der vorherigen Ansprüche sowie ein Wundversorgungssystem zur Wunddrainage unter Einsatz von Unterdruck auf.

Ebenso kann der erfindungsgemäße Wundpflegeartikel auch in ein Wundversorgungssystem zur Wunddrainage unter Einsatz von Unterdruck eingebracht sein.

Solche Systeme sind z.B. in den Schriften DE202004017052, WO2006048246 und DE202004018245 der Anmelderin der vorliegenden Erfindung offenbart.

Aus erstgenannter ist eine Vorrichtung zur Wundbehandlung unter Einsatz von Unterdruck bekannt, aufweisend ein gasdichtes Wundabdeckungselement, das im am Körper des Patienten angelegten Zustand einen zwischen der jeweiligen Wunde und dem Wundabdeckungselement verbleibenden Raum bildet, und wenigstens eine Anschlussstelle, die mit dem Raum in Kontakt steht und über welche die im Raum befindliche Luft evakuiert werden kann, wobei das Wundabdeckungselement von wenigstens einer flächenhaften, die Wundexsudate aufnehmenden Wundauflage unterlegt ist, dessen Volumen im Laufe des Absorptionsprozesses zunimmt, so dass die absorbierten Wundexsudate innerhalb der Wundauflage und damit unterhalb des Wundabdeckungselementes bis zur Entfernung der Wundauflage aus dem Körper des Patienten verbleiben, die Wundauflage wenigstens eine Lage eines mit Superabsorbentien angereicherten Textilabschnittes ist, die mit einer flüssigkeitsdurchlässigen Hülle umgeben ist, und die Lage in Draufsicht auf ihre Flachseite eine Fläche hat, die 3% bis 90% kleiner als die der Hülle ist, damit sich die Wundauflage in der Nähe seiner gesamten Füllungskapazität im Querschnitt einer Kreisform annähern kann.

Aus zweitgenannter ist ein Mehrkomponentenverband zur Wundbehandlung des menschlichen oder tierischen Körpers unter Einsatz von Unterdruck bekannt, aufweisend: ein Wundabdeckungselement zur Anbringung an Haut- und Schleimhautoberfläche, wenigstens eine Anschlussstelle, die mit dem Wundraum in Kontakt steht und über welche die im Wundraum befindlichen Stoffe evakuiert werden können, wobei dieser superabsorbierende Polymere aufweist, wobei die absorbierten Wundexsudate an Polymere gebunden im Wundraum bis zur Entfernung aus dem Wundraum verbleiben, wobei die Polymere durch ihre Bindungskapazität wechselseitige Synergien mit den subatmosphärischen Drücken unterstützen.

Aus letztgenannter ist eine Drainagevorrichtung zur Wundbehandlung unter Einsatz von Unterdruck bekannt, aufweisend ein gasdichtes, aus folienartigem Material bestehendes Wundabdeckungselement, das im am Körper des Patienten angelegten Zustand an der Hautoberfläche um den Wundbereich herum adhäsiv befestigt ist und einen zwischen der jeweiligen Wunde und dem Wundabdeckungselement verbleibenden, abgedichteten Raum bildet, wenigstens einen Drainageschlauch, der in den Raum einsetzbar ist, über den die im Raum befindlichen Stoffe evakuiert werden können, und wenigstens eine innerhalb des Raumes angeordnete, die Wundexsudate aufsaugende Wundauflage, die wenigstens eine Lageeines mit Superabsorbentien angereicherten Textilabschnittes aufweist, die mit einer flüssigkeitsdurchlässigen Hülle umgeben ist, wobei die absorbierten Wundexsudate innerhalb der Wundauflage und damit unterhalb des Wundabdeckungselementes bis zur Entfernung der Wundauflage aus dem Körper des Patienten verbleiben, und wobei das Wundabdeckungselement eine gasdicht verschliessbare Behandlungsöffnung aufweist, durch die die Wundauflage in den Raum einlegbar und aus dem Raum entnehmbar ist.

Der erfindungsgemäße Wundpflegeartikel kann überdies eine an anatomische Gegebenheiten angepasste Form aufweisen. Hierzu kann er z.B. in Form einer Manschette ausgebildet sein; die über den einen Arm oder ein Bein oder ein Gelenk gestülpt werden kann, oder in Form eines an die Ferse, das Ellenbogengelenk oder dergleichen angepassten Verbandes.

Dabei erweist sich eine Kombination mit Mikrofasern als besonders vorteilhaft, da Mikro fasern aufgrund ihrer polsternden Wirkung die Maske des aufliegenden Unterdrucksystems angenehm abpolstern. Überdies unterstützen Mikrofasern aufgrund ihrer feuchtigkeitsleitenden Eigenschaften den Drainageprozess besonders effektiv. Besonders vorteilhaft ist dabei die Verwendung eines wie oben beschriebenen Abstandsgewirkes, - gestricks oder -geleges aufweisend Mikrofasern.

So ist zum Beispiel ein Unterdrucksystem zur gelenknahen Anbringung, zum Beispiel zur Anbringung am Ellenbogen oder der Ferse denkbar, wie es von der Firma KCI, San Antonio USA, auf dem Markt ist, wobei der bei diesem Produkt verwendete Polyurethanschaum durch die erfindungsgemäße Mikrofaser mit polsternder Wirkung ersetzt ist. Das Ersetzen des bekannten Polyurethanschaums durch die erfindungsgemäße Mikrofaser stellt eine kostengünstige Alternative gegenüber dem bekannten Material dar und bietet darüber hinaus gegenüber der Struktur des Polyurethanschaums ein zielgerichteteres Ableiten der Wundflüssigkeiten durch eine gerichtete Ausgestaltung der Mikrofasern

Erfindungsgemäß ist ferner der Wundpflegeartikel gemäß einem der Ansprüche 1 bis 14 zur Verwendung in der Kontrolle von Exsudat, zur Polsterung einer Wunde und/oder zur Zerstörung eines Biofilms vorgesehen.

Erfindungsgemäß ist ebenso der Wundpflegeartikel gemäß einem der Ansprüche 1 bis 14 oder ein Kit gemäß Anspruch 15 oder 16 zur Verwendung in der Behandlung von chronischen, akuten und/oder blutenden Wunden, Verbrennungswunden und/oder traumatisch erzeugten Wunden vorgesehen.

Weitere Ausführungsformen:
Vorzugsweise besteht eine ggf. vorgesehene Hülle zumindest teilweise auch aus einem hydrophobem Material, beispielsweise aus Polypropylen oder aus einem hydrophob ausgerüsteten Naturmaterial, wie Baumwolle. Die hydrophoben Eigenschaften der Hülle verhindern das Verkleben mit der Wundoberfläche und tragen dazu bei, dass das Wundexsudat schneller ins Innere der Hülle gelangen kann.

Die Hülle kann auch aus einem anderen Kunststoff, insbesondere einer Polyurethan- oder Polyethylenfolie oder aus künstlicher Spinnenseiden Folie hergestellt sein.

Das Material der Hülle kann derart strukturiert sein, dass die Hülle eine raue Außenfläche und eine glatte Innenfläche aufweist. Vorzugsweise ist die raue Außenfläche der Hülle durch trichterförmige Perforationen gebildet, die sich jeweils in Richtung Innenfläche verjüngen und in eine freie Öffnungskante ("Auskragung") auslaufen. Dementsprechend kann die glatte Innenfläche des Hüllenmaterials durch gewölbte, sich zwischen den Perforationen erstreckende Materialabschnitte gebildet sein. Ein solches Hüllenmaterial kann im Gegensatz zu einem beidseitig ebenen als "dreidimensional" bezeichnet werden, und ist z.B. aus der DE102006017194 der Anmelderin der vorliegenden Anmeldung in umgekehrter Weise (glatte Außenfläche, rauhe Innenfläche) bekannt.

Bevorzugt ist überdies vorgesehen, dass die Hülle eines erfindungsgemäßen Wundpflegartikels zumindest abschnittsweise eine adhäsive Beschichtung aufweist, und zwar bevorzugt auf der wundabgewandten Seite, mit deren Hilfe sie - z.B. mit einem Verband - im Wundbereich fixiert werden kann.

Weiterhin kann auch vorgesehen sein, dass die Hülle einen über die eigentliche Wunde überstehenden Bereich aufweist, der mit Klebestreifen zur Fixierung versehen ist. Überdies kann auch auf der wundabgewandten Seite der Wundauflage ein flächiger Materialabschnitt vorgesehen sein, der über die eigentliche Hülle hinausgeht und wenigstens in seinen Randbereichen eine der Haut zugewandte adhäsive Beschichtung z.B. in Form von Klebestreifen oder -flächen aufweist (sog. "Island Dressing").

Besagter flächiger Materialabschnitt kann insbesondere semiokklusive bzw. semipermeable Eigenschaften aufweisen, d.h. er kann z.B. durchlässig für Feuchtigkeit sein, nicht aber für Bakterien.

Die Permeabilität für Wasserdampf liegt dabei bevorzugt im Bereich zwischen > 500 g bis < 16000 g m⁻² h ¹.

Als Klebematerialien für die oben genannten Zwecke kommen bevorzugt physiologisch akzeptable adhäsive Agenzien in Frage, wie z.B. Hydrokolloidkleber oder medizinisch unbedenkliche Klebstoffe, wie lösungsmittelfreie, biokompatible Silikonkleber oder Polyacrylatkleber sein, die eine gute Beständigkeit gegen alle gängigen Sterilisationsverfahren aufweisen.

Der Polyurethanschaum lässt sich im Gegensatz zu anderen Kunststoffen, wie Polypropylen, Polytetrafluorethylen (Teflon) und Silikon gut kleben.

In einer besonderen Ausführungsform ist vorgesehen, dass der absorbierende Körper innerhalb der Hülle unsymmetrisch, d.h. überwiegend auf einer Seite angeordnet ist.

Ein solcher Wundpflegeartikel lässt sich bevorzugt in Wundtaschen einsetzen, in denen beengte Raumverhältnisse herrschen. Dabei verbleibt der Abschnitt des Wundpflegeartikels, in dem sich ein absorbierender Körper befindet, außerhalb der Wundtasche. Durch die Kapillarkräfte werden Exsudate, die sich in einer solchen Wundtasche befinden, effektiv aufgenommen und auch hier die Wundheilung gefördert.

Bevorzugt ist weiterhin vorgesehen, dass der Wundpflegeartikel einen lateralen Einschnitt oder eine keilförmige Aussparung aufweist, dergestalt, dass sich die Ränder des Einschnitts bzw. der Aussparung überlappend anordnen lassen.

Bevorzugt ist weiterhin vorgesehen, dass der Wundpflegeartikel einen die Wunde ausfüllenden Abschnitt aufweist. Dieser kann z.B. so ausgestaltet sein, dass er bei Kontakt mit Exsudat aufquillt und die Wunde bis zum Wundboden ausfüllt.

Auf diese Weise ist der Wundpflegeartikel dreidimensional formbar, so dass sich eine konkave Form ergibt, um ihn z.B. für die Auflage an ein Gelenk, eine Extremität oder ein gekrümmtes Körperteil anzupassen. Die überlappend angeordneten Ränder des Einschnitts bzw. der Aussparung können dabei z.B. durch Klettverschlüsse, Druckköpfe, Klebestreifen oder andere geeignete Fixierungsmittel fixiert werden.

In einer anderen bevorzugten Ausgestaltung ist vorgesehen, dass der Wundpflegeartikel dreidimensional geformt ist, dergestalt, dass er für die Auflage an ein Gelenk, eine Extremität oder ein gekrümmtes Körperteil angepasst ist.

So kann der Wundpflegeartikel z.B. eine konkave Form aufweisen, um ihn z.B. für die Auflage an die Ferse eines Fußes oder an einen Ellenbogen eines Patienten anzupassen. Der absorbierende Körper, der im Inneren dieser so geformten Wundauflage angeordnet ist, kann dabei herausnehmbar gestaltet sein. Besonders von Vorteil ist diese Ausgestaltung, wenn der absorbierende Körper zuvor angefeuchtet wurde bzw. durch austretendes Exsudat angefeuchtet wird, da sich hier dann aufgrund der vorliegenden superabsorbierenden Polymere ein Gel ausbildet, das polsternd wirkt und somit die schmerzfreie Lagerung des besagten Körperteils ermöglicht. Überdies passt sich die Wundauflage durch die Anfeuchtung noch besser den anatomischen Gegebenheiten an.

Bevorzugt ist überdies vorgesehen, dass der Wundpflegeartikel zumindest teilweise in einer gerollten Form vorliegt. Hier kann vorgesehen sein, dass der ursprünglich flächige Wundpflegeartikel gerollt und ggf. in ihrer gerollten Form z.B. durch Nähen, Kleben oder Schweissen fixiert wird. Ein solcher Wundpflegeartikel eignet sich insbesondere für die Verwendung als Tamponade in Wundtaschen; sie weist insbesondere eine Dochtfunktion für die aufzunehmenden Exsudate auf.

Bevorzugt ist in diesem Zusammenhang außerdem vorgesehen, dass der Wundpflegeartikel mindestens einen nutritiven, mindestens einen desinfizierenden bzw. dekontaminierenden und/oder mindestens einen Proteasen hemmend wirkenden Wirkstoff und/oder Wirkstoffkomplex aufweist.

Besagter Wirkstoff kann z.B. in getrockneter Form in die abrasiv wirkende Matrix eingearbeitet sein, wo er bei Kontakt mit Flüssigkeit (z.B. Exsudat) gelöst wird und in die Wunde gelangt.

Bei dem desinfizierend wirkenden Wirkstoff und/oder Wirkstoffkomplex kann es sich z.B. um eine Zusammensetzung aus mindestens einem Vitamin oder Vitaminderivat, einem Metallion sowie einem Detergenz handeln. Ebenso kann es sich dabei um ein BLIS (bacteriocin like inhibitory substance), um ein antimikrobielles Peptid, ein Antibiotikum, ein Silberpräparat oder um beschichtete magnetische Partikel handeln. Besonders bevorzugt sind hier quaternäre Ammoniumverbindungen zu nennen.

Bei dem nutritiv wirkenden Wirkstoff und/oder Wirkstoff komplex kann es sich um eine Zusammensetzung enthaltend mindestens die Bestandteile eines enteralen und/oder parenteralen Diätetikums handeln. Ebenso kann es sich dabei um mindestens ein Wirkelement ausgewählt aus der Gruppe enthaltend Insulin, rekombinantes Insulin, Proinsulin, einen insulinähnlichen Wachstumsfaktor (Insulin-like growth factor, IGF), ein Insulinmimetikum und/oder einen diabetikerspezifischen, nicht glucose- bzw. saccharosebasierenden Energieträger handeln.

Genauso kann es sich bei dem nutritiv wirkenden Wirkstoff und/oder Wirkstoff komplex um ein Glycolyse-Enzym handeln, so z.B. um eine Hexokinase, die Glucose in Glucose-6-Phosphat überführt und so durch Einleitung der Glycolyse den bei Diabetikern häufig erhöhten Glucosespiegel reduziert.

Bei dem Proteasen hemmend wirkenden Wirkstoff und/oder Wirkstoff komplex kann es sich um mindestens ein Wirkelement ausgewählt aus der Gruppe enthaltend Proteasehemmer, superabsorbierende Polymere, Antikörper gegen Matrix-MetalloProteasen (MMP, insbesondere gegen MMP 2, 7 und 9), Chelatoren für zweiwertige Kationen (insbesondere für Ca²⁺) , Kollagen, beschichtete magnetische Partikel, Säuren, Puffer, nicht pathogene säureproduzierende Mikroorganismen, Probiotika und/oder Symbiotika handeln. Insbesondere kann dabei vorgesehen sein, dass in der Wundauflage eine Trägersubstanz vorgesehen ist, die zweiwertige Kationen (insbesondere Ca²⁺) bindet.

Weitere Zusammenhänge und Hintergründe zu den nutritiven, einen desinfizierenden bzw. dekontaminierenden und/oder Proteasen hemmend wirkenden Wirkstoffen und/oder Wirkstoffkomplexen sind in der DE 102007030931 der Anmelderin der vorliegenden Anmeldung beschrieben. In der DE 102007030931 sind auch weitere nutritive, desinfizierende bzw. dekontaminierende und/oder Proteasen hemmend wirkende Wirkstoffen und/oder Wirkstoffkomplexe beschrieben.

Weiterhin kann der Wundpflegeartikel eine Zubereitung enthaltend Phagen und/oder Bestandteile derselben enthalten. Eine solche Wundauflage ist in der DE102007054127 der Anmelderin der vorliegenden Erfindung beschrieben.

Es kann überdies vorgesehen sein, dass der Wundpflegeartikel nekrolytische und/oder fibrinolytische Enzyme enthält. Ebenso kann sie Angiogenese- oder Epidermogenesefördernde Wachstumsfaktoren enthalten (insbesondere aus der Gruppe der VEGF und EGF). Ebenso kann der Wundpflegeartikel Lockstoffe für Makrophagen enthalten, die die bei der Phagentherapie freiwerdenden Phagen und Bakterienreste (insbesondere Endotoxine) phagocytieren.

Weiterhin ist bevorzugt vorgesehen, dass mindestens ein Abschnitt der Hüllenwand des Wundpflegeartikels ein Reservoir für mindestens einen nutritiven, einen desinfizierenden bzw. dekontaminierenden, einen Proteasen hemmend wirkenden, einen blutstillend wirkenden und/oder einen wundheilungsfördernden Wirkstoff und/oder Wirkstoffkomplex aufweist.

Besagtes Reservoir kann z.B. aus einer in die Hüllenwand eingearbeiteten Tasche bestehen. Ebenso kann das Reservoir aus einem mit besagtem Wirkstoff und/oder Wirkstoff komplex getränkten Abschnitt der Hüllenwand bestehen, oder der Wirkstoff ist in besagten Abschnitt eingepresst.

Dem Wundpflegeartikel können Substanzen zugefügt sein, die den osmotischen Druck erhöhen können. Zu den Substanzen zählen z. B. Osmodiuretika, wie Mannitol.

Mineralische Ionenaustauscher, wie Zeolithe, Bentonite oder Montmarylinite, können ebenfalls Bestandteil der Wundauflage, insbesondere seiner Matte sein. Zeolithe können u. a. Schadstoffe, wie Schwermetalle, absorbieren. Überdies entfalten sie eine blutstillende Wirkung.

Ebenso kann der Artikel auch Aktivkohle enthalten. Diese ist bevorzugt in einer dünnen Lage, beispielsweise in einer Vliesschicht, dispergiert, auf der wundabgewandten Seite angeordnet, und dient insbesondere dazu, unangenehme Gerüche aus dem Wundbereich zu absorbieren.

Weiterhin kann der Wundpflegeartikel eine Zubereitung enthaltend Phagen und/oder Bestandteile derselben enthalten. Eine solche Wundauflage ist in der DE102007054127 der Anmelderin der vorliegenden Erfindung beschrieben.

Der erfindungsgemäße Wundpflegeartikel kann außerdem so ausgebildet sein, dass er sich zur Umlage um eine chirurgisch angelegte Leitung eignet. Hierzu kann der Wundpflegeartikel z.B. wenigstens einen Schlitz aufweisen, der es ermöglicht, die Wundauflage am Körper eines Patienten um eine Leitung (z.B. eine Drainageleitung oder einen Katheter) umzulegen. Ein solcher Wundpflegeartikel ist z.B. aus der DE202006005966 der Anmelderin der vorliegenden Erfindung bekannt. Dabei kann im distalen Bereich des Schlitzes ein Steg, ein Knopf, eine Kettelnaht, eine Schweißnaht, eine perforierte Brücke oder eine andere lösbare Verbindung ("Sollbruchstelle") vorgesehen sein, die es ermöglicht, den Wundpflegeartikel in der gewohnten Art und Weise oder in der Art einer Schlitzkompresse zu verwenden.

Hier ist besonders wichtig, dass der erfindungsgemäße Wundpflegeartikel bei Flüssigkeitsaufnahme nicht anschwillt und an Volumen zunimmt, da auf diese Weise verhindert wird, dass die umgebene Leitung verengt oder blockiert wird.

Ebenso ist in diesem Zusammenhang bevorzugt vorgesehen, dass der Wundpflegeartikel mindestens ein Agenz aufweist, das die Blutung oder die Blutungsneigung einschränken kann.

Bei besagtem Agens kann es sich um mindestens einen chemisch und/oder physiologisch wirkenden Wirkstoff bzw. Wirkstoff komplex oder um mindestens ein physikalisch wirkendes Wirkelement handeln. Eine solcher Wundauflage ist z.B. aus der DE 10 2007 030 931 der Anmelderin der vorliegenden Anmeldung bekannt.

Hierzu kann der Wundpflegeartikel beispielsweise
- als im Wesentlichen flacher Materialabschnitt aufweisend Absorptionsmaterial, der aus einem aufsaugenden Vlies mit darin verteilten superabsorbierenden Polymeren sowie mindestens einem chemisch und/oder physiologisch wirkende Wirkstoff bzw. Wirkstoffkomplex ausgebildet sein,
- als oder in Kombination mit einem Druck- oder Kompressionsverband, insbesondere als Teil einer Kompressionstherapie bei Ulcus cruris venosum
- als eine Kombination aus einem primären, nicht oder nur unwesentlich absorbierenden Wundpflegeartikel, der mindestens einen chemisch und/oder physiologisch wirkenden Wirkstoff bzw. Wirkstoffkomplex aufweist, und einer peripher von diesem primären Wundpflegeartikel angeordneten sekundären Wundpflegeartikel, der superabsorbierende Polymere enthält, wobei ggf. zwischen beiden eine Diffusionsbarriere angeordnet ist,
- in Form eines Verbandpäckchens, aufweisend einen primären Wundpflegeartikel mit mindestens einem chemisch und/oder physiologisch wirkenden Wirkstoff bzw. Wirkstoffkomplex sowie einem an dem Wundpflegeartikel angeordneten Wickelabschnitt, der zumindest abschnittsweise superabsorbierende Polymere aufweist, und/oder
- als Materialabschnitt mit einer Längserstreckung aufweisend Absorptionsmaterial, wobei der Materialabschnitt elastisch verformbare Eigenschaften aufweist, und wobei der Materialabschnitt superabsorbierende Polymere sowie ggf. mindestens einen chemisch und/oder physiologisch wirkende Wirkstoff bzw. Wirkstoffkomplex aufweist.

Bevorzugt handelt es sich bei dem chemisch und/oder physiologisch wirkenden Wirkstoff bzw. Wirkstoff komplex um mindestens einen Stoff bzw. eine Zusammensetzung, die Blutstillende Eigenschaften aufweist. Diese Stoffe sind unter dem Oberbegriff "Hämostatika" bekannt.

Bevorzugt handelt es sich bei dem chemisch und/oder physiologisch wirkenden Wirkstoff bzw. Wirkstoff komplex um mindestens einen Stoff bzw. eine Zusammensetzung, die Blutstillende Eigenschaften aufweist. Diese Stoffe sind unter dem Oberbegriff "Hämostatika" bekannt.

Bei dem physikalisch wirkenden Wirkelement handelt es sich z.B. um eine Abbindung, ein Druckpolster, einen Druckverband oder einen Kompressionsverband.

### Definitionen

Der Begriff "Wundpflegeartikel" bezeichnet, neben klassischen primären und sekundären Wundauflagen, auch Wundpflegetücher, Wundreinigungspads, Wundreinigungstücher, Tupfer, Tampons, Wundtamponaden und dergleichen. Diese können absorbierende und nicht absorbierende Eigenschaften haben.

Der Begriff "Exsudat" bezeichnet eine über die entzündlichen Prozesse des Wundödems vom Blutplasma abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen hohen Gehalt an Wachstumsfaktoren und Zytokinen.

Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert, wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert irreversibel geschädigtes Gewebe abbauen und somit das Wundbett für die nachfolgenden Phasen der Heilung vorbereiten.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Unter dem Begriff "chemisch und/oder physiologisch wirkenden Wirkstoff bzw. Wirkstoffkomplex" sollen im Folgenden solche Wirkstoffe bzw. Wirkstoffkomplexe verstanden werden, die die Blutung oder die Blutungsneigung einzuschränken imstande sind, ohne dass dabei physikalische Kräfte angewendet werden müssen. Der Wirkungsweg ist hier eine chemische und/oder physiologische Interaktion mit dem Wundmilieu.

Unter dem Begriff "Wirkstoffkomplex" soll im Folgenden nicht nur ein Komplex im chemischen Sinne verstanden werden, sondern insbesondere eine Zusammensetzung synergistisch eine Wirkung hervorrufender Wirkstoffe.

Unter dem Begriff "physikalisch wirkendes Wirkelement" soll im Folgenden ein Wirkelement verstanden werden, das auf physikalischem Wege, d.h. durch die Ausübung von Druck, Zug, Kälte und dergleichen, die Blutung oder die Blutungsneigung einzuschränken imstande ist.

Unter dem Begriff "Abbindung" soll im Folgenden eine notfallmedizinische Maßnahme verstanden werden, die in der Lage ist, den arteriellen Blutfluß z.B. in einer Gliedmaße zu stoppen, um so einen unvertretbaren Blutverlust in einer Wunde zu verhindern. Indikation für eine solche Abbindung sind in der Regel traumatische Einwirkungen, die zu einer Verletzungen mindestens einer Arterie führen.

Unter dem Begriff "chronische Wunden" sollen Wunden verstanden werden, die nicht primär auf traumatische Einwirkungen zurückgehen. Zwar können traumatische Einwirkungen der ursprüngliche Auslöser einer solchen Wunde gewesen sein, aber die chronische Wunde zeichnet sich vor allem durch eine verzögerte Wundheilung auf. Chronische Wunden weisen häufig - wenn überhaupt - nur leichte Blutungen auf, dafür oftmals eine starke Exsudation.

Unter dem Begriff "leichte Blutung" soll eine Blutung verstanden werden, die nicht arteriellen Ursprungs ist, sondern ggf. venösen Ursprungs oder interstitiellen bzw. kapillaren Ursprungs, und die in jedem Fall so leicht ausfällt, dass sie nicht mittel- oder unmittelbar lebensbedrohend ist.

Unter dem Begriff "akut blutende Wunden" sollen solche Wunden verstanden werden, die zu großen Blutverlusten führen. In der Regel sind hierfür arterielle Blutungen verantwortlich, die z.B. durch traumatische Einwirkungen verursacht werden. Akut blutende Wunden können u.U. mittel- oder unmittelbar lebensbedrohend sein. Aus diesem Grunde hat bei akut blutenden Wunden die Blutungsstillung eine sehr hohe Priorität.

Unter dem Begriff "Druckverband" soll im Folgenden der aus der Notfallmedizin bekannte Druckverband verstanden werden, Dieser besteht aus einem nicht zu harten, nicht saugfähigen Gegenstand (Druckpolster) ohne scharfe oder harte Kanten, der auf eine bereits abgedeckte Wunde aufgebracht wird und mithilfe einer Wickel mit mäßigem Zug befestigt wird. Durch den ausgeübten Druck wird die Durchblutung des betreffenden Körperteils gemindert und die traumatisch geöffneten Blutgefäße werden wieder geschlossen.

Unter dem Begriff "nicht oder nur unwesentlich absorbierende Wundauflage" soll eine Wundauflage bezeichnen, die ein geringes Absorptionsvermögen für Flüssigkeiten aufweist. Insgesamt soll das Absorptionsvermögen dabei bei weniger als 60 Gew.-%, bevorzugt weniger als 20 Gew.-% des Trockengewichts der Wundauflage liegen. Primäre Aufgabe einer solchen Wundauflage ist daher auch nicht die Aufnahme von Blut oder Exsudaten, sondern die Abgabe blutstillender Agenzien im Sinne der vorliegenden Erfindung.

Unter dem Begriff "Kompressionsverband" wird hingegen in aller Regel ein Verband verstanden, der ähnlich wie ein Druckverband wirkt, jedoch auf das genannte Druckpolster verzichtet. Der Druck oder die Kompression auf die Wunde wird hierbei ausschließlich durch die Wickel ausgeübt. Hierbei kann das Wickelmaterial elastisch sein.

Der erfindungsgemäße Wundpflegeartikel eignet sich aufgrund der oben genannten Eigenschaften insbesondere für die Behandlung mit traumatischer, thermischer, chemischer, durch ionisierende Strahlung bedingter Genese.

### Zeichnungen und Beispiele

Die vorliegende Erfindung wird durch die im Folgenden gezeigten und diskutierten Figuren und Beispiele genauer erläutert. Dabei ist zu beachten, dass die Figuren und Beispiele nur beschreibenden Charakter haben und nicht dazu gedacht sind, die Erfindung in irgendeiner Form einzuschränken. Figuren 1-7 und 14-20 sind nicht erfindungsgemäss.

Die Figuren zeigen:
- Fig.1: einen flachen, dreischichtigen Wundpflegeartikel in einer perspektivischen Ansicht;
- Fig. 2: den Wundpflegeartikel gemäß Fig. 1 in einem schematischen Schnitt;
- Fig. 3: einen anderen dreischichtigen Wundpflegeartikel, in einem schematischen Schnitt;
- Fig. 4: ein vergrößertes Detail des Wundpflegeartikels gemäß Fig. 3;
- Fig. 5: einen umhüllten Absorptionskörper, in einem schematischen Schnitt;
- Fig. 6: einen zweischichtigen Wundpflegeartikel mit einer umlaufenden Naht, in einem schematischen Schnitt;
- Fig. 7: einen Wundpflegeartikel mit einem Träger, in einem schematischen Schnitt;
- Fig. 8: einen anderen Wundpflegeartikel mit Träger, in einem schematischen Schnitt;
- Fig. 9: einen florartigen Wundpflegeartikel, ebenfalls in einem schematischen Schnitt;
- Fig. 10: einen anderen umhüllten Absorptionskörper, mit Zellstoff-Flocken, in einem schematischen Schnitt;
- Fig. 11: einen weiteren erfindungsgemäßen Wundpflegeartikel;
- Fig. 12: das erfindungsgemäße Wirkprinzip;
- Fig. 13: die rasterelektronenmikroskopische Aufnahme eines Biofilms;
- Fig. 14 - 16: unterschiedliche Ausgestaltungen eines erfindungsgemäßen Wundpflegeartikels aufweisend einen flächigen Absorptionskörper mit einem Muster aus Inzisionen
- Fig. 17-20: unterschiedliche Ausgestaltungen eines erfindungsgemäßen Wundpflegeartikels in Form eines Flüssigkeitsdurchlässigen Primärverbands in Bahnenform, aufweisend Perforationen, die einen Flüssigkeitsdurchtritt ermöglichen und sich Wundseite hin öffnen um so die besprochene rauhe, abrasiv wirkende Oberfläche auszubilden.

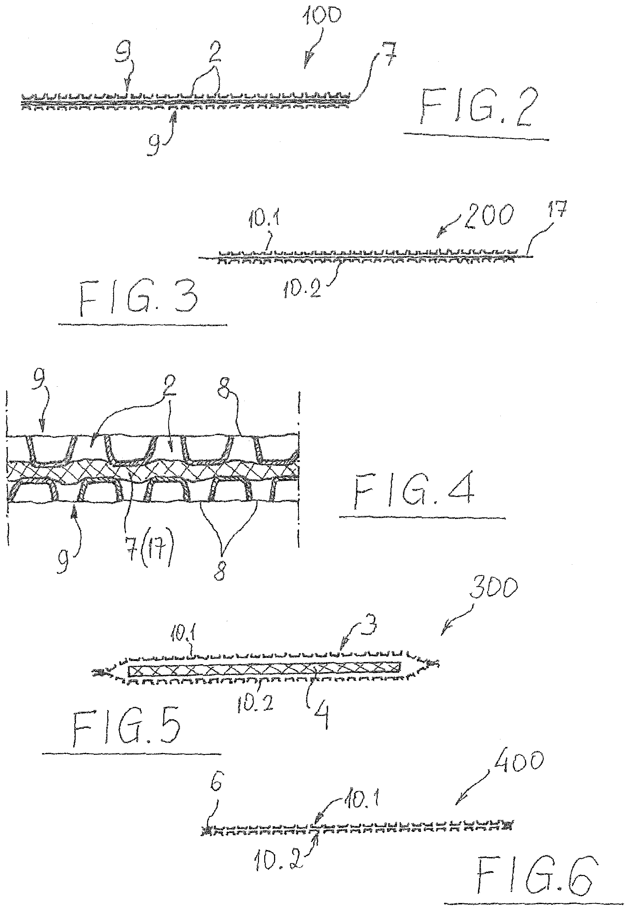

In Figuren 1 und 2 ist ein sandwichartiger Wundpflegeartikel 100 dargestellt, der sich aus zwei zueinander spiegelsymmetrisch angeordneten Materialabschnitten 10.1, 10.2 und einem dazwischen liegenden, netzartigen Träger 7 zusammensetzt. Die Materialabschnitte 10.1, 10.2 und der Träger 7 sind rechteckig und deckungsgleich. Die Symmetrie des Wundpflegeartikels bezieht sich auf die Anordnung der beiden Materialabschnitte 10.1, 10.2 gegenüber dem Träger 7.

Die Materialabschnitte 10.1, 10.2 sind jeweils aus mit Silikon beschichteter, etwa 30 µm dicker Polyethylenfolie hergestellt, die eine Vielzahl von Perforationen 2 aufweist. Diese Folie entspricht in Ihrer Aufmachung z.B. einer Folie aufweisend eine dreidimensionale Porenstruktur, wie sie z.B. in der WO2007118652 der Anmelderin der vorliegenden Erfindung beschrieben ist. Die etwa trichterförmigen Perforationen 2 weisen jeweils eine nach außen gerichtete Öffnungskante 8 auf (vgl. Fig. 4) und bilden dadurch eine erste, rauhe Oberfläche 9. Eine zweite, um die Perforationen verminderte Oberfläche 13 (vgl. Fig. 1) des Materialabschnittes 10.1, 10.2 bleibt glatt.

Der Träger 7 besteht aus Polyesternetz von Maschenweite etwa 0,3 bis 0,5 mm, das beidseitig mit medizinischem, dickflüssigem Paraffin durchtränkt ist. Die Materialabschnitte 10.1, 10.2 sind mit ihren glatten Oberfläche 13 dem Träger 7 zugewandt und mit diesem über das Paraffin lösbar verklebt. Da die Materialabschnitte 10.1, 10.2 silikonbeschichtet sind und mit ihren glatten Oberfläche 13 mit dem Paraffin kontaktieren, können sie an der dünnen Paraffinschicht gleiten. Diese Eigenschaft des Wundpflegeartikels 100 prädestiniert ihn zur Verwendung als reversibel Primärverband, dessen eine, erste raue Oberfläche 9 direkt auf eine mit Biofilm bedeckte, chronische Wunde aufgelegt werden kann und dessen andere raue Oberfläche es erlaubt, mit der ersten Oberfläche 9 eine scheuernde Bewegung per Hand auszuüben, die jedoch durch die Paraffinschicht etwas gemildert wird. Durch die scheuernde Bewegung wird der Biofilm zerstört und die Wundoberfläche zur Exsudation angeregt. Der als Primärverband verwendete Wundpflegeartikel 100 kann mit einem Sekundärverband, beispielsweise mit einem Verbandmull (nicht dargestellt) abgedeckt und am Körper des Patienten mittels eines Klebebandes fixiert sein. Da die beiden Flachseiten des Wundpflegeartikels 100 miteinander identisch sind, kann dieser mit beliebiger Oberfläche auf die Wunde gelegt werden, wodurch die Arbeit des Fachpersonals erleichtert werden kann.

Die Figuren 3 und 4 zeigen einen flachen Wundpflegeartikel 200, bestehend aus den bereits beschriebenen Materialabschnitten 10.1, 10.2, die miteinander über eine hauchdünne Vaselin-Schicht 17 lösbar verbunden sind. Das Vaselin ist alkali-und säurefrei und entspricht den Reinheitsanforderungen des Deutschen Arzneibuches. Wie in Fig. 4 deutlich zu sehen ist, sind die folienartigen Materialabschnitte 10.1, 10.2 ebenfalls gleitend an der mittleren Vaselin-Schicht 17 angeordnet.

In Fig. 6 ist ein einfachster Wundpflegeartikel 400 gezeigt, aufweisend zwei in der oben erwähnten Anordnung aufeinander gelegte, rechteckige Materialabschnitte 10.1, 10.2, die miteinander über eine umlaufende, periphere Schweißnaht 6 verbunden sind. Es ist keine mittlere, zwischen den Materialabschnitten 10.1, 10.2 liegende Klebeschicht vorgesehen. Der besagte Wundpflegeartikel 400 ist ebenfalls zum Auflegen auf den Biofilm und dessen Unterbrechung bzw. Zerstörung bestimmt.

Der Fig. 5 ist ein Wundpflegeartikel 300 zu entnehmen, der eine Weiterentwicklung des in Fig. 6 gezeigten Wundpflegeartikels 400 darstellt. Die beiden peripher beispielsweise über Ultraschallnähte miteinander verbundenen Materialabschnitte 10.1, 10.2 bilden eine flüssigkeitsdurchlässige Hülle 3, in der ein flacher Absorptionskörper 4 untergebracht ist. Der Absorptionskörper 4 besteht aus offenzelligem, die Wundexsudate aufzunehmenden Polyurethan-Weichschaum. Optional kann der schaumstoffartige Absorptionskörper 4 superabsorbierende Substanzen, vornehmlich in Granulat- oder Pulverform, aufweisen. Dementsprechend kann mittels Wundpflegeartikels 300 der bestehende Biofilm zerstört und das nach dem Entfernen von Biofilm entstehende Wundexsudat durch den Absorptionskörper 4 aufgesaugt werden. Das überschüssige Wundexsudat kann durch weitere, nach dem Entfernen des besagten Wundpflegeartikels 300 auf die Wunde auflegbare andere, an sich bekannte Absorptionskörper bzw. Wundkompressen angesammelt werden.

Abweichend von der Darstellung in Fig. 5 können die Nähte (beispielsweise Ultraschallnähte) auch nach innen gekehrt sein (die Hülle also quasi "auf links gezogen" vorliegen), um weiche und für den Wundkontakt angenehme Kanten auszubilden.

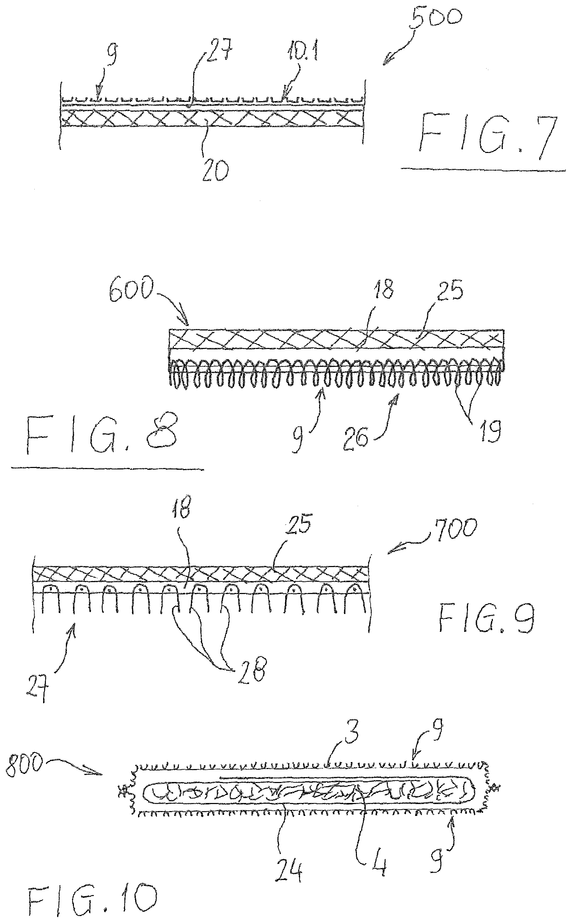

Gemäß Fig. 10 besteht der Absorptionskörper 4 aus einer etwa 4 mm dicken Schicht von Zellstoff-Flocken, die mit superabsorbierenden Fasern vermengt und von einer inneren, papierartigen Umhüllung 24 umgeben sind. Der Gewichtsanteil an superabsorbierenden Fasern in der Zellstoffschicht beträgt 17%. Die beschriebenen Teile sind in der äußeren, flüssigkeitsdurchlässigen Hülle 3 untergebracht und bilden zusammen mit der Hülle einen kissenförmigen, polsternden Wundpflegeartikel 800. Die Funktionsweise des Wundpflegeartikels 800 ist der des Wundpflegeartikels 300 gleich. Schließlich kann der Absorptionskörper 4 aus wenigstens einer Lage einer an sich bekannten Airlaid-Matte gefertigt sein, die vorzugsweise mit oben erwähnten superabsorbierenden Substanzen angereichert ist.

Die Fig. 7 zeigt einen ausschließlich für die Zerstörung von Bio film bestimmten Wundpflegeartikel 500, bestehend aus einer etwa 4 mm dicken Trägerschicht 20 aus Polyurethan-Weichschaum, einem silikonbeschichteten Materialabschnitt 10.1 und einer dazwischen angeordneten Paraffinschicht 27. Der Materialabschnitt 10.1 ist mit seiner rauen Oberfläche 9 nach außen gerichtet. In einer weiteren, nicht dargestellten Ausführungsform sind auf der Trägerschicht 20 zwei Materialabschnitte 10.1, 10.2 aufkaschiert, so dass der Wundpflegeartikel mit seiner beliebigen rauhen Flachseite auf die Wunde gelegt werden kann.

In Fig. 8 ist ein Wundpflegeartikel 600 dargestellt, bestehend aus einem Grundgewebe 18 und aus in das Grundgewebe mit eingewebten Schlingen 19 sowie aus einem Träger 25 aus Polyurethanschaum. Der Träger 25 ist auf das Grundgewebe 18 aufkaschiert. Sowohl das Grundgewebe 18 als auch die Schlingen 19 bestehen aus einer Mischung aus Polyester-und Polyamid-Mikrofasern von einem Titer 0,8 dtex. Der Anteil an Polyamid-Mikrofasern beträgt etwa 20%. Die Schlingen 19 bilden eine Bürste 26, mit der der bestehende Biofilm an der Wunde abgescheuert werden kann.

Die Fig. 9 zeigt einen ebenso für das Abtragen eines Biofilms bestimmten Wundpflegeartikel 700, bei dem in das Grundgewebe 18 anstelle der Schlingen (vgl. Fig. 8) Polfäden 28 zum Einsatz kommen, die florartig über das Grundgewebe ragen und ebenfalls eine Bürste 27 bilden. Die Polfäden 28 sind auf bekannter Weise im Grundgewebe verankert. Beim Gebrauch kratzen die Polfäden 28 den Biofilm an der Wunde und nehmen auch Teil des Biofilms auf.

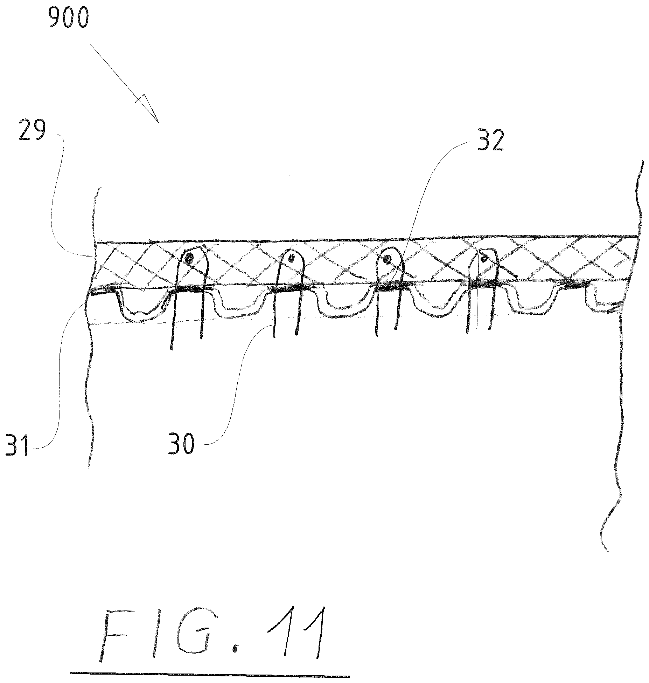

Die Fig. 11 zeigt einen weiteren erfindungsgemäßen Wundpflegeartikel 900, bestehend aus einer Schicht 29 aufweisend ein Gewebe mit Polfäden (Flor) 30, sowie aus einer Schicht 31 bestehend aus einer Folie aufweisend eine dreidimensionale Porenstruktur. Diese Folie entspricht in Ihrer Aufmachung z.B. einer Folie aufweisend eine dreidimensionale Porenstruktur, wie sie z.B. in der WO2007118652 der Anmelderin der vorliegenden Erfindung beschrieben ist. Die etwa trichterförmigen Perforationen 32 weisen entgegen z.B. der in Fig. 1 gezeigten Anordnung von der Wunde weg, wobei die Polfäden 30 durch die Perforationen hindurch zur Wunde hin weisen, und bei Anordnung des Wundpflegartikels auf einer Wunde eine abrasive Wirkung auf den Biofilm in der Wunde ausüben.

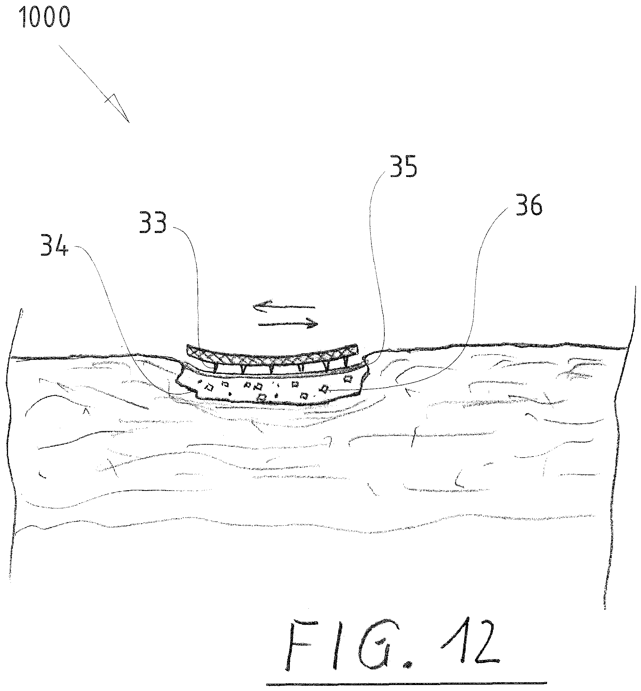

Die Fig. 12 zeigt beispielhaft das Wirkprinzip eines Erfindungsgemäßen Wundpflegeartikels 1000, der über abrasive Wirkelemente 33 verfügt, wie sie in der vorliegenden Anmeldung beschrieben sind. Durch die durch die Pfeile angedeuteten Relativbewegungen des erfindungsgemäßen Wundpflegeartikels zur Wunde 34 üben die abrasiven Wirkelemente eine abrasive Wirkung auf den Bio film 35 aus und entfernen diesen. Dabei werden durch den Biofilm geschützte Bakterien 36 freigelegt, die entweder ebenfalls durch die abrasive Wirkung entfernt werden oder aber anschließend mit Desinfektionsmitteln oder Antibiotika entfernt werden können.

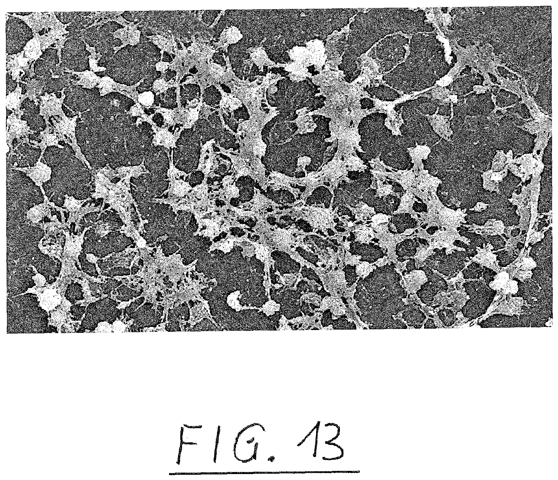

Die Fig. 13 zeigt eine rasterelektronenmikroskopische Aufnahme eines Biofilms aufweisend den Eitererreger Staphylococcus aureus. Deutlich sind zwischen den einzelnen Bakterien (Kokken) die Querbrückenartigen Abschnitte des Biofilms zu erkennen, der u. A. aus sauren Mucopolysaccharidschichten besteht und eine Art Schutzschild ausbildet, unter welchem die Bakterien gedeihen.

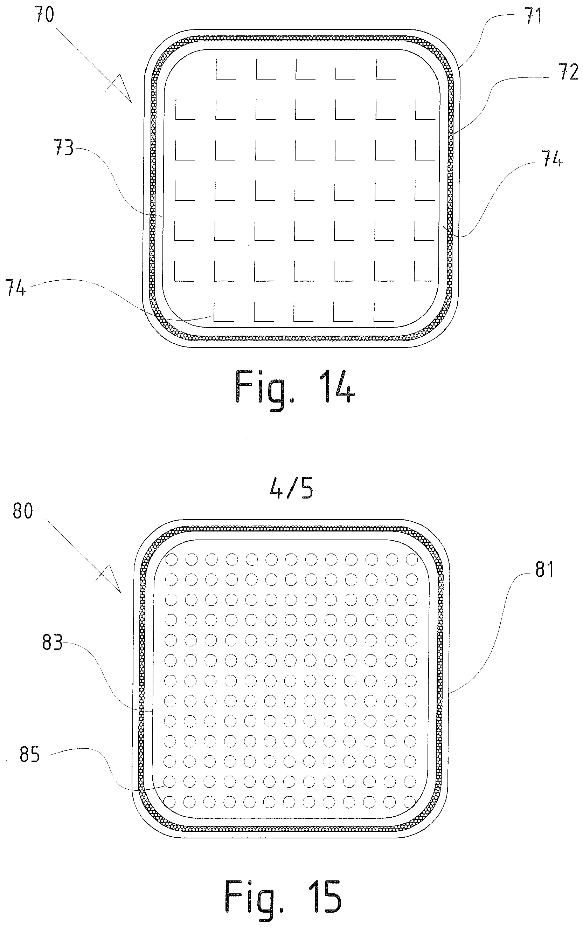

Fig. 14 zeigt einen Wundpflegeartikel 70 in Draufsicht, aufweisend eine Hülle 71 mit einer Naht 72, die aus einem dreidimensionalen Folienmaterial mit nach außen gekehrten Öffnungen bzw. Perforationen besteht, die dem Wundpflegeartikel eine rauhe Außenfläche und damit abrasive Eigenschaften verleihen, sowie einem flächigen Absorptionskörper 73 aus einem Vlies- oder Airlaidmaterial enthaltend superabsorbierende Polymere. Die Hülle bildet einen Expansionsraum 74 aus, sodass gewährleistet ist, dass der Absorptionskörper bei Flüssigkeitsaufnahme in seinem Volumen zunehmen kann und nicht durch die Hülle eingeschränkt ist. Der flächige Absorptionskörper 73 weist ein Muster aus L-Förmigen Inzisionen 75 auf, die mittels eines entsprechend ausgestalteten Stanzwerkzeugs in den Absorptionskörper eingebracht sind. Auf diese Weise wird der Flüssigkeitseintritt in den Wundpflegeartikel wesentlich erleichtert. Dieses Merkmal entfaltet besondere Vorteile im Zusammenspiel mit der Hülle aus einem dreidimensionalen Folienmaterial mit nach außen gekehrten Öffnungen bzw. Perforationen, die dem Wundpflegeartikel eine rauhe Außenfläche und damit abrasive Eigenschaften verleihen.

Fig. 15 zeigt einen weiteren Wundpflegeartikel 80 in Draufsicht, aufweisend eine ähnlich wie in Fig. 14 gestaltete Hülle 81 sowie einen flächigen Absorptionskörper 83 aus einem Vlies- oder Airlaidmaterial enthaltend superabsorbierende Polymere. Der flächige Absorptionskörper 83 weist ein flächiges Muster aus Lochstanzungen 85 auf, die mittels eines entsprechend ausgestalteten Stanzwerkzeugs in den Absorptionskörper eingebracht sind. Auf diese Weise wird der Flüssigkeitseintritt in den Wundpflegeartikel erleichtert. Ferner wird die Anschmiegsamkeit des ursprünglich relativ steifen Absorptionskörpers erhöht, so dass ein Wundpflegeartikel erzielt wird, der sich sanft an das Wundrelief anschmiegt, vom Patienten als sehr weich und angenehm empfunden wird und durch den engen Kontakt zur Wunde seine Wundexsudate aufnehmende Funktion voll entfalten kann.

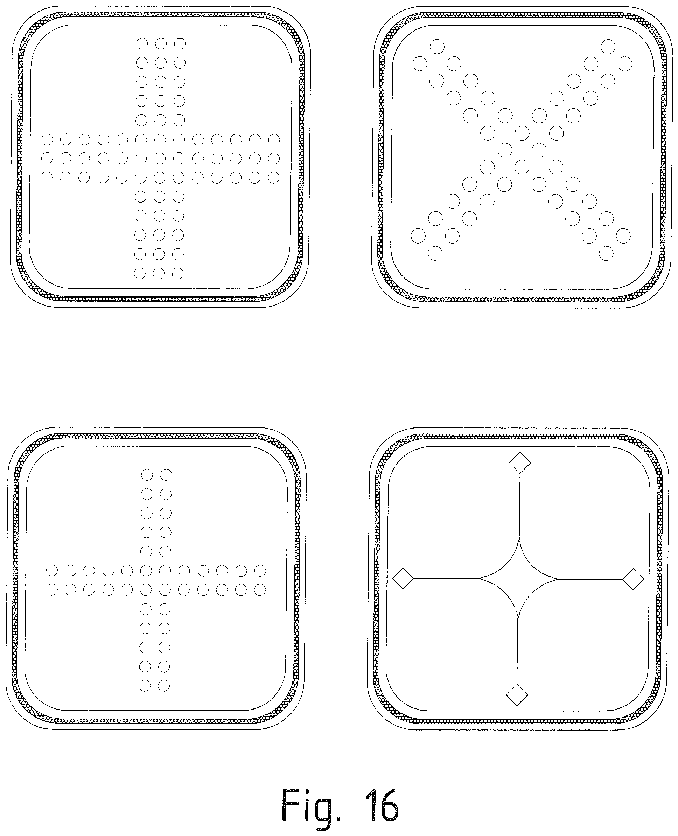

Fig. 16 zeigt weitere Ausgestaltungen des erfindungsgemäßen Wundpflegeartikels, wobei die Stanzungen und/oder Inzisionen, die teils gemeinsam in einem Wundpflegeartikel verwirklicht sind, den Flüssigkeitseintritt in den Wundpflegeartikel erleichtern.

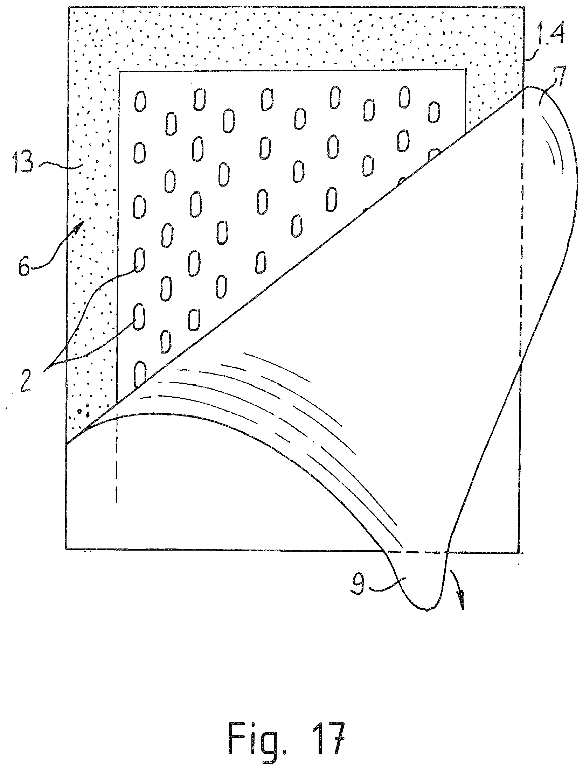

Fig. 17 zeigt einen erfindungsgemäßen Wundpflegeartikel in Form eines Flüssigkeitsdurchlässigen Primärverbands in Bahnenform, aufweisend Perforationen, die einen Flüssigkeitsdurchtritt ermöglichen. Die Perforationen öffnen sich zur Wundseite hin und bilden so die besprochene rauhe, abrasiv wirkende Oberfläche aus. Der Flüssigkeitsdurchlässige Primärverband in Bahnenform kann teils oder vollständig als Hülle für einen Wundpflegeartikel aufweisend einen absorbierenden Körper agieren.

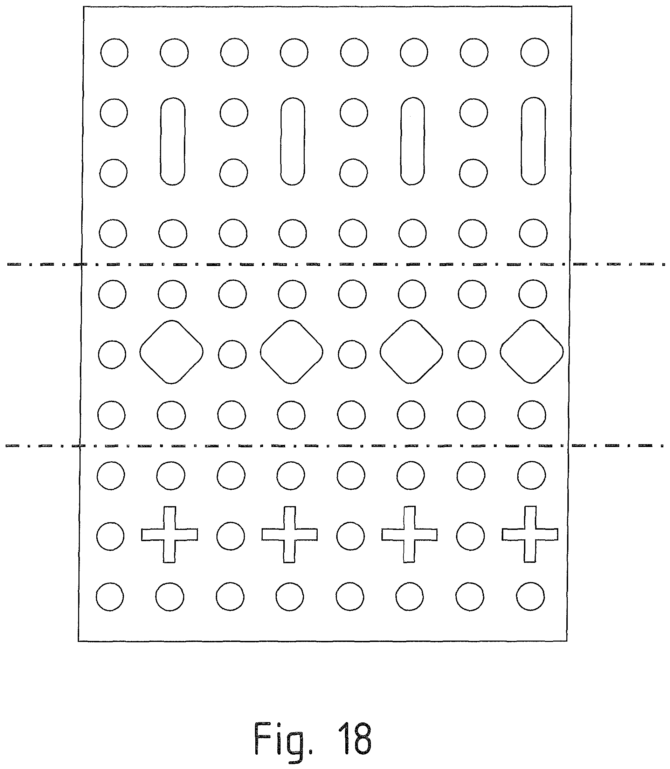

Fig. 18 zeigt einen ähnlichen Wundpflegeartikel in Form eines Flüssigkeitsdurchlässigen Primärverbands in Bahnenform, aufweisend Perforationen, die einen Flüssigkeitsdurchtritt ermöglichen. Auch hier öffnen sich die Perforationen sich zur Wundseite hin und bilden so die besprochene rauhe, abrasiv wirkende Oberfläche aus. Der Flüssigkeitsdurchlässige Primärverband in Bahnenform kann teils oder vollständig als Hülle für einen Wundpflegeartikel aufweisend einen absorbierenden Körper agieren. Ferner weist dieser Wundpflegeartikel Stanzungen, Schlitze, Inzisionen und/oder Ausnehmungen beispielsweise in Form von Langlöchern, Karos und/oder oder Kreuzen auf, die einem erleichterten Flüssigkeitsdurchtritt dienen.

Dies ist besonders dann vorteilhaft, wenn das Material aus einem dreidimensionalen Folienmaterial mit nach außen bzw. zur Wunde gekehrten Öffnungen bzw. Perforationen besteht, die dem Wundpflegeartikel eine rauhe Außenfläche und damit abrasive Eigenschaften verleihen. Durch die abrasiven Eigenschaften wird die Exsudation der Wunde angeregt, und es kann zu Flüssigkeitsansammlungen im oberen Wundbereich kommen, die abgeleitet werden müssen. Die genannten Stanzungen, Schlitze, Inzisionen und/oder Ausnehmungen gewährleisten einen erleichterten Durchtritt sowie eine effektive und schnelle Aufnahme des Exsudats, dass durch Verwendung des erfindungsgemäßen Wundpflegeartikels erzeugt wird.

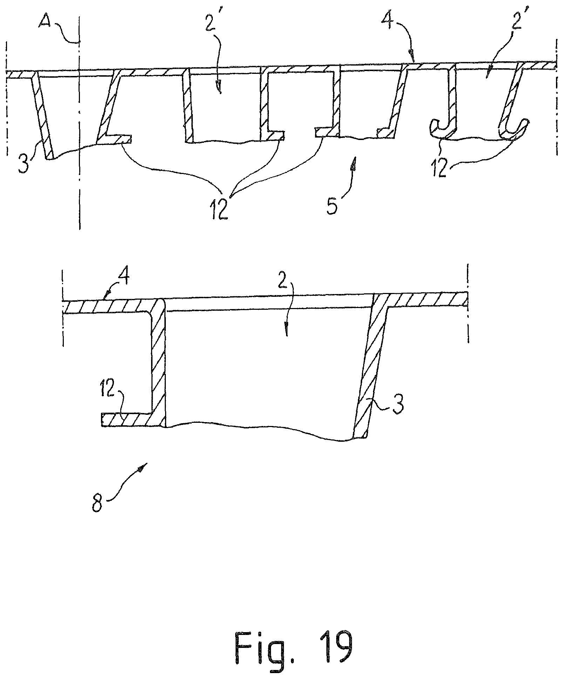

Fig. 19 zeigt die besprochenen Perforationen im Querschnitt. Dabei ist erkennbar, dass letztere konisch ausgebildete Wandungen besitzen, welche wiederum unregelmäßig in etwa senkrecht zu einer Perforationsachse A gerichtete Auskragungen auslaufen. Diese Auskragungen können auch nach innen oder nach außen umgekrempelt sein, wie es die rechte Seite der Fig. 19 zeigt. Die beschriebenen Auskragungen sind für die abrasive Wirkung nicht unbedingt erforderlich.

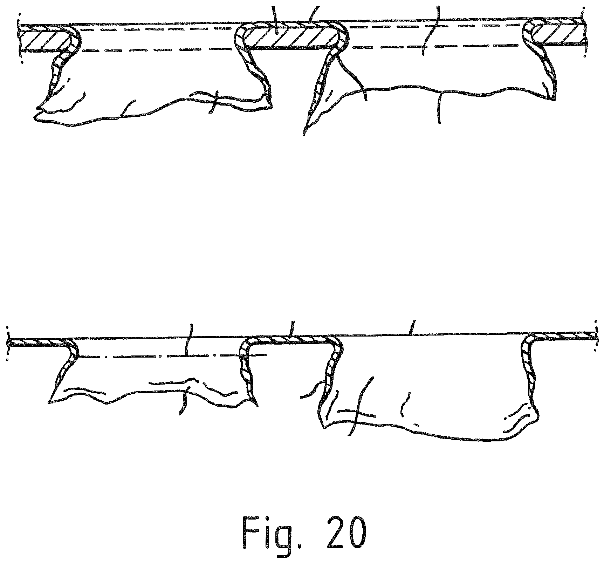

Fig. 20 zeigt ebenfalls die besprochenen Perforationen im Querschnitt, diesmal in einer naturalistischen Darstellung. Hier ist zu erkennen, dass die in Fig. 19 beschriebenen Auskragungen nicht vorkommen.

## Patentansprüche

1. Wundpflegeartikel, aufweisend mindestens eine Oberfläche mit abrasiven Eigenschaften, die so ausgebildet ist, dass der Wundpflegeartikel geeignet ist, bei Relativbewegung desselben zu einer Wunde
a) in der Wunde angeordnete Biofilme aufzubrechen, und/oder
b) die Wundexsudation anzuregen,
**dadurch gekennzeichnet, dass** die mindestens eine Oberfläche mit abrasiven Eigenschaften im Wesentlichen schräg oder senkrecht zur Wundoberfläche angeordnete bzw. anordnungsfähige Mikrofasern aufweist,
wobei die Mikrofasern direkt in Kontakt mit der Wunde kommen können und der Flüssigkeiten absorbierende Abschnitt in einer zweiten Schicht angeordnet ist.

2. Wundpflegeartikel gemäß Anspruch 1, **dadurch gekennzeichnet, dass** der Wundpflegeartikel als Wundauflage, Wundreinigungspad, Wundreinigungstuch, Tupfer, Tampon oder Wundtamponade ausgeformt ist.

3. Wundpflegeartikel gemäß Anspruch 1 oder 2, **dadurch gekennzeichnet, dass** die Mikrofasern mindestens einen Fasertyp aufweisen, der ausgewählt ist aus der Gruppe enthaltend
• Polyesterfasern, wie z.B Trevira Finesse, Diolen^{®} Soft, Fortrel Microspun, DuPont Micromattique, Primabelle und/oder Shingosen,
• Polyamidfasern, wie Nylon, Timbrelle^{®}, Supplex Microfiber, Tactel^{®} Micro und/oder Silky Touch,
• Acrylfasern, wie Microsupreme, und/oder
• Polyurethanfasern.

4. Wundpflegeartikel nach einem der vorhergehenden Ansprüche, **dadurch gekennzeichnet, dass** der Wundpflegeartikel einen Materialabschnitt mit abrasiven Eigenschaften aufweist, der gegenüber dem übrigen Wundpflegeartikel frei schwimmend angeordnet ist, dergestalt, dass er im auf den Körper des Patienten aufgelegten Zustand seine Lage gegenüber dem übrigen Wundpflegeartikel bzw. gegenüber der Wunde ändern kann, wobei der Materialabschnitt bevorzugt in einer viskosen Schicht schwimmt.

5. Wundpflegeartikel gemäß einem der vorhergehenden Ansprüche, **dadurch gekennzeichnet, dass** der Wundpflegeartikel einen Wundexsudate absorbierenden Körper und eine mindestens abschnittsweise um diesen Körper angeordnete Hülle aufweist.

6. Wundpflegeartikel gemäß Anspruch 5, **dadurch gekennzeichnet, dass** der Wundpflegeartikel, insbesondere der Wundexsudate absorbierende Körper, superabsorbierende Polymere aufweist, wobei die die superabsorbierenden Polymere bevorzugt eine Pulver-, Granulat-, Faser-, Garn-, Watte-, Vlies-, und/oder Gewebeform aufweisen.

7. Wundpflegeartikel gemäß Anspruch 5 oder 6, **dadurch gekennzeichnet, dass** der Wundexsudate absorbierende Körper ein Muster aus Inzisionen und/oder Stanzungen aufweist, wobei die Inzisionen und/oder Stanzungen bevorzugt dergestalt ausgebildet und/oder angeordnet sind, dass sie den Flüssigkeitseintritt in den Wundpflegeartikel erleichtern, wobei die Letztgenannten insbesondere im Zusammenspiel mit einer Hülle aus einem dreidimensionalen Folienmaterial mit nach außen bzw. zur Wunde gekehrten Öffnungen bzw. Perforationen vorkommen, die dem Wundpflegeartikel eine rauhe Außenfläche und damit abrasive Eigenschaften verleihen.

8. Wundpflegeartikel gemäß Anspruch 6-7, **dadurch gekennzeichnet, dass** die verwendeten superabsorbierenden Polymere im Wundpflegeartikel immobilisiert sind, wobei die Immobilisierung bevorzugt auf eine der folgenden Arten erfolgt:
a) die superabsorbierenden Polymere mit Fasern der Wundauflage verklebt sind, z.B. mit einem Hotmelt-Kleber;
b) die superabsorbierenden Polymere mit Fasern der Wundauflage verpresst sind;
c) die superabsorbierenden Polymere fest in eine dreidimensionale Fasermatrix eingebettet sind;
d) der die superabsorbierenden Polymere enthaltende Abschnitt der Wundauflage von einer Hülle umgegeben ist, die - z.B. durch Wahl einer geeigneten Porengröße oder durch Verzicht auf Poren - undurchlässig ist für die Polymere ist;
e) die superabsorbierenden Polymere in Faserform vorliegen, wobei die Fasern in Form eines Faserverbundes vorliegen und ggf. sogar mit Zellstofffasern der Wundauflage einen Verbund eingehen.

9. Wundpflegeartikel gemäß einem der vorhergehenden Ansprüche, **dadurch gekennzeichnet, dass** der Wundpflegeartikel weiterhin aufweist:
• einen Abschnitt aufweisend Weichschaumstoff
• einen Abschnitt aufweisend modifizierte Cellulose, insbesondere Carboxymethylcellulose, und/oder
• einen Abschnitt aufweisend Alginate, und/oder
• einen Abschnitt aufweisend ein Vlies und/oder eine Airlaidmatte, oder Schnipsel derselben.

10. Wundpflegeartikel gemäß Anspruch 5 - 9, **dadurch gekennzeichnet, dass** zwischen der Hülle und dem Wundexsudate absorbierenden Körper wenigstens ein flüssigkeitsundurchlässiger Wäscheschutz-Folienabschnitt angeordnet ist.

11. Wundpflegeartikel gemäß einem der vorhergehenden Ansprüche, **dadurch gekennzeichnet, dass** das Material aufweisend eine Oberfläche mit abrasiven Eigenschaften darüber hinaus Stanzungen, Schlitze, Inzisionen und/oder Ausnehmungen aufweist, die einem erleichterten Flüssigkeitsdurchtritt dienen.

12. Kit für die Akut-, Notfall- oder Militärmedizinische bzw. die chronische Versorgung, aufweisend einen Wundpflegeartikel gemäß einem der Ansprüche 1 bis 11.

13. Kit gemäß Anspruch 12, aufweisend einen Wundpflegeartikel gemäß einem der Ansprüche 1 bis 11 sowie ein Wundversorgungssystem zur Wunddrainage unter Einsatz von Unterdruck.

14. Wundpflegeartikel gemäß einem der Ansprüche 1 bis 11 zur Kontrolle von Exsudat, zur Polsterung einer Wunde und/oder zur Zerstörung eines Biofilms.

15. Wundpflegeartikel gemäß einem der Ansprüche 1 bis 11 oder Kit gemäß Anspruch 12 oder 13 zur Behandlung von chronischen, akuten und/oder blutenden Wunden, Verbrennungswunden und/oder traumatisch erzeugten Wunden.

## Claims

1. Wound care article, comprising at least one surface having abrasive properties, which is embodied such that the wound care article is suitable, in case of relative movement of the same with respect to a wound, to
a) break up biofilms arranged in the wound, and/or
b) excite wound exudation;
**characterized in that** the at least one surface with abrasive properties has microfibers which are or can be arranged substantially at an incline or perpendicular to the wound surface,
where the microfibers can come in direct contact with the wound and the liquid absorbing portion is arranged in a second layer.

2. Wound care article according to Claim 1, **characterized by** being embodied as a wound dressing, wound cleaning pad, wound cleaning tissue, swab, plug or wound tamponade.

3. Wound care article according to Claim 1 or 2, **characterized in that** the microfibers contain at least one type of fiber selected from the group containing
- polyester fibers, such as e. g. Trevira Finesse, Diolen^{®} Soft, Fortrel Microspun, DuPont Micromattique, Primabelle and/or Shingosen,
- polyamide fibers, such as nylon, Timbrelle^{®}, Supplex Microfiber, Tactel^{®} Micro and/or Silky Touch,
- acrylic fibers, such as Microsupreme, and/or
- polyurethane fibers.

4. Wound care article according to one of the above Claims, **characterized by** having a material portion with abrasive properties which is arranged free-floating with respect to the rest of the wound care article, such that in the state in which it is applied on the patient's body, it can change its position with respect to the rest of the wound care article or with respect to the wound, respectively; the material portion preferably floating in a viscous layer.

5. Wound care article according to one of the above Claims, **characterized in that** the wound care article has a body absorbing wound exudates and an envelope arranged at least in portions around this body.

6. Wound care article according to Claim 5, **characterized in that** the wound care article, in particular the body absorbing wound exudates, contains superabsorbent polymers which preferably have the form of a powder, granulate, fiber, yarn, cotton wool, fleece and/or tissue.

7. Wound care article according to Claim 5 or 6, **characterized in that** the body absorbing wound exudates has a pattern consisting of incisions and/or punchings, which are preferably embodied and/or arranged such that they facilitate the penetration of liquid into the wound care article, the latter being provided in particular in combination with an envelope made of a three-dimensional film material with openings or perforations directed outwards or to the wound, respectively, which provide the wound care article with a rough outer surface and thus with abrasive properties.

8. Wound care article according to Claims 6-7, **characterized in that** the employed superabsorbent polymers are immobilized in the wound care article, immobilization preferably occurring in one of the following ways:
a) the superabsorbent polymers are adhesively bonded to fibers of the wound dressing, e. g. by means of a hotmelt adhesive;
b) the superabsorbent polymers are compressed with fibers of the wound dressing;
c) the superabsorbent polymers are firmly embedded in a three-dimensional fiber matrix;
d) the portion of the wound dressing containing the superabsorbent polymers is surrounded by an envelope which- e. g. by selection of a suitable pore size or by omitting the pores - is impermeable to the polymers;
e) the superabsorbent polymers are present in the form of fibers, the fibers being present in the form of a fiber composite and, if applicable, even forming a composite with pulp fibers of the wound dressing.

9. Wound care article according to one of the above Claims, **characterized in that** the wound care article further comprises:
- a portion containing flexible foam,
- a portion containing modified cellulose, in particular carboxymethylcellulose, and/or
- a portion containing alginates and/or
- a portion containing a fleece and/or an airlaid mat, or shreds of the same.

10. Wound care article according to Claims 5-9, **characterized in that** at least one liquid-impermeable film portion for clothing protection is arranged between the envelope and the body absorbing the wound exudates.

11. Wound care article according to one of the above Claims, **characterized in that** the material having a surface with abrasive properties in addition has punchings, slits, incisions and/or recesses which serve to facilitate liquid passage.

12. Kit for acute, emergency or military medical care or chronical care, respectively, containing a wound care article according to one of Claims 1 through 11.

13. Kit according to Claim 12, containing a wound care article according to one of Claims 1 through 11 as well as a wound care system for wound drainage by means of negative pressure.

14. Wound care article according to one of Claims 1 through 11 for controlling exudate, for padding a wound and/or for destroying a biofilm.

15. Wound care article according to one of Claims 1 through 11, or kit according to Claim 12 or 13, for treatment of chronical, acute and/or bleeding wounds, burn injuries and/or traumatically caused wounds.

## Revendications

1. Article pour le soin des plaies présentant au moins une surface aux propriétés abrasives, qui est conçu pour que l'article pour le soin des plaies soit approprié, lors d'un mouvement relatif de celui-ci avec une plaie pour
a) casser des biofilms disposés dans la plaie, et/ou
b) provoquer l'exsudation de la plaie,
**caractérisé en ce que** l'au moins une surface aux propriétés abrasives présente essentiellement des microfibres disposées, respectivement pouvant être disposées de manière oblique ou verticale à la surface de la plaie,
dans lequel les microfibres peuvent venir directement en contact avec la plaie et le tronçon absorbant les liquides est disposé dans une seconde couche.

2. Article pour le soin des plaies selon la revendication 1, **caractérisé en ce que** l'article pour le soin des plaies est formé en tant que gaze pour plaie, tampons de nettoyage de plaie, lingette de nettoyage de plaie, compresses ou tampon de plaie.

3. Article pour le soin des plaies selon la revendication 1 ou 2, **caractérisé en ce que** les microfibres présentent au moins un type de fibre qui est sélectionné parmi le groupe comprenant
• des fibres de polyester comme par exemple Trevira Finesse, Dionlen^{®} Soft, Fortrel Microspun, DupPont Micromattique, Primabelle et/ou Shingosen,
• des fibres de polyamide comme le nylon, Timbrelle^{®}, Supplex Microfiber, Tactel^{®} Micro et/ou Silky Touch,
• des fibres acryliques, comme Microsupreme, et/ou
• des fibres de polyuréthane.

4. Article pour le soin des plaies selon l'une des revendications précédentes, **caractérisé en ce que** l'article pour le soin des plaies présente un tronçon de matériau aux propriétés abrasives, qui est disposé baignant librement par rapport au reste de l'article pour le soin des plaies, de telle façon que dans l'état où il est posé sur le corps du patient, il peut modifier sa position par rapport au reste de l'article pour le soin des plaies, respectivement par rapport à la plaie, dans lequel le tronçon de matériau baigne de préférence dans une couche visqueuse.

5. Article pour le soin des plaies selon l'une des revendications précédentes, **caractérisé en ce que** l'article pour le soin des plaies présente un corps absorbant les exsudats de plaie et une enveloppe disposée autour de ce corps au moins par tronçons.

6. Article pour le soin des plaies selon la revendication 5, **caractérisé en ce que** l'article pour le soin des plaies, en particulier le corps absorbant les exsudats de plaie présente des polymères super-absorbants, dans lequel les polymères super-absorbants présentent de préférence une forme de poudre, granulat, fibres, fils, ouate, non-tissé et/ou de tissé.

7. Article pour le soin des plaies selon la revendication 5 ou 6, **caractérisé en ce que** le corps absorbant les exsudats de plaie présente un motif fait d'incisions et/ou de poinçonnages, dans lequel les incisions et/ou poinçonnages sont de préférence formés et/ou disposés de façon à ce qu'ils facilitent la pénétration du liquide dans l'article pour le soin des plaies, dans lequel ces derniers, notamment en coopération avec une enveloppe de matériau en film tridimensionnel, se présentent avec des ouvertures, respectivement perforation, dirigées vers l'extérieur, respectivement vers la plaie, qui confèrent à l'article pour le soin des plaies une surface extérieure rugueuse et donc des propriétés abrasives.

8. Article pour le soin des plaies selon la revendication 6-7, **caractérisé en ce que** les polymères super-absorbants employés sont immobilisés dans l'article pour le soin des plaies, dans lequel l'immobilisation s'effectue de préférence d'une des manières suivantes:
a) les polymères super-absorbants sont collés à des fibres de la gaze pour plaie, par exemple avec une colle par fusion à chaud;
b) les polymères super-absorbants sont comprimés avec des fibres de la gaze pour plaie;
c) les polymères super-absorbants sont enchâssés fixement dans une matrice de fibres tridimensionnelle;
d) le tronçon de la gaze pour plaie comprenant les polymères super-absorbants est entouré par une enveloppe qui est imperméable aux polymères, par exemple en choisissant une grosseur de pore appropriée ou en renonçant aux pores;
e) les polymères super-absorbants sont présents sous forme de fibre, dans lequel les fibres sont présents sous forme de fibres composites, et composent un composite avec des fibres de cellulose de la gaze pour plaie.

9. Article pour le soin des plaies selon l'une des revendications précédentes, **caractérisé en ce que** l'article pour le soin des plaies présente en outre
• un tronçon présentant une mousse souple
• un tronçon présentant de la cellulose modifiée, en particulier de la carboxyméthylcellulose, et/ou
• un tronçon présentant de l'alginate, et/ou
• un tronçon présentant un non-tissé et/ou un tapis Airlaid ou des petits morceaux de celui-ci.

10. Article pour le soin des plaies selon les revendications 5 - 9, **caractérisé en ce qu'**au moins un tronçon de film de protection du linge imperméable aux fluides est disposé entre l'enveloppe et le corps absorbant l'exsudat de plaie.

11. Article pour le soin des plaies selon l'une des revendications précédentes, **caractérisé en ce que** le matériau présentant une surface aux propriétés abrasives présente en outre des poinçonnages, des fentes, des incisions et/ou des évidements qui servent à faciliter la pénétration du liquide.

12. Set pour la médecine intensive, d'urgence ou militaire, respectivement le traitement de maladies chroniques, présentant un article pour le soin des plaies selon l'une des revendications 1 à 11.

13. Set selon la revendication 12, présentant un article pour le soin des plaies selon l'une des revendications 1 à 11 ainsi qu'un système de traitement de la plaie pour le drainage de la plaie par l'emploi de dépression.

14. Article pour le soin des plaies selon l'une des revendications 1 à 11 destiné à contrôler l'exsudat, à garnir une plaie et/ou à détruire un biofilm.

15. Article pour le soin des plaies selon l'une des revendications 1 à 11 ou set selon la revendication 12 ou 13 pour le traitement de plaies chroniques, graves et/ou en sang, des plaies de brûlures et/ou traumatiques.
